# EUROPEAN PATENT SPECIFICATION

(11) **EP 4 547 180 B1**
(45) Date of publication and mention of the grant of the patent: **07.10.2026**
(21) Application number: 23744332.0
(22) Date of filing: 27.06.2023
(51) Int. Cl.: A61F 13/49, A61F 13/496

(54) **ABSORBENT ARTICLES WITH FRANGIBLE PATHWAYS AND CONCEALED DISPOSAL FASTENER COMPONENTS**
ABSORBIERENDE ARTIKEL MIT ZERBRECHLICHEN PFADEN UND VERBORGENEN ENTSORGUNGSBEFESTIGUNGSELEMENTEN
ARTICLES ABSORBANTS À VOIES FRANGIBLES ET COMPOSANTS DE FIXATION À ÉLIMINATION DISSIMULÉE

(30) Priority: 30.06.2022 US 202263357043 P; 14.12.2022 US 202263432400 P; 14.12.2022 US 202263432401 P; 14.12.2022 US 202263432402 P; 14.12.2022 US 202263432403 P; 14.12.2022 US 202263432404 P; 14.12.2022 US 202263432406 P; 14.12.2022 US 202263432410 P; 14.12.2022 US 202263432413 P
(43) Date of publication of application: 07.05.2025
(73) Proprietor: The Procter & Gamble Company, Cincinnati, OH 45202 (US)
(72) Inventor: RAYCHECK, Jeromy, Thomas, Cincinnati, Ohio 45202 (US); WILLHAUS, Keith, Richard, Cincinnati, Ohio 45202 (US); ROSIAK, Jeffry, Cincinnati, Ohio 45202 (US); NAYLOR, Jason, Edward, Cincinnati, Ohio 45202 (US); CHATTERJEE, Kumardipti, Cincinnati, Ohio 45202 (US); LONG, Michael, Devin, Cincinnati, Ohio 45202 (US); TAYLOR, Nicholas, Alexander, Cincinnati, Ohio 45202 (US)
(74) Representative: P&G Patent Germany
(86) International application number: PCT/US2023/069117
(87) International publication number: WO 2024/006719

(56) References cited:
- US-A1- 2009 312 734
- US-A1- 2017 105 883
- US-A1- 2019 209 392
- US-B1- 6 508 797
- US-B2- 7 150 730
- US-B2- 7 527 617
- US-B2- 8 118 799
- US-B2- 9 226 861

## Description

### FIELD OF THE INVENTION

The present disclosure relates to absorbent articles, and more particularly, to absorbent articles having front and/or back waist regions including one or more frangible pathways with concealed disposal fastener components.

### BACKGROUND OF THE INVENTION

Some absorbent articles have components that include elastomeric laminates. Such elastomeric laminates may include an elastic material bonded to one or more nonwovens. The elastic material may include an elastic film and/or elastic strands. In some laminates, a plurality of elastic strands are joined to a nonwoven while the plurality of strands are in a stretched condition so that when the elastic strands relax, the nonwoven gathers, and in turn, forms corrugations and rugosities. The resulting elastomeric laminate is stretchable to the extent that the corrugations allow the elastic strands to elongate.

Absorbent articles in the form of diaper pants may also be configured with an absorbent chassis connected with front and back elastic belts, wherein opposing end regions of the front and back belts are connected with each other at side seams. In some instances, the elasticity of the front and back belts is removed in regions where the chassis connects with the belts. Thus, in some converting configurations adapted to assemble such diaper pants, stretched elastic strands are glued between two continuous nonwoven webs to form an elastic laminate. Regions of the elastic strands may then be intermittently deactivated along the length of the elastic laminate by cutting the elastic strands in areas to be connected with the chassis, sometimes referred to as tummy elastic cutting.

Some caregivers of older incontinent babies or toddlers may prefer a closed, pant-style disposable absorbent article to enable application to, and removal from, a child while the child is in a standing position. One disadvantage of this product form is that the removal and disposal of feces-containing products may be unhygienic and inconvenient. For example, pulling the product down could cause feces to smear down the legs of a user. In other examples, a caregiver may tear open the bonded sides using force. In turn, the force used can lead to a rapid release of energy from the diaper, causing the caregiver to lose control of the product and allowing feces to spill out. In contrast, removal and disposal of traditional open or taped diaper forms with fasteners may be readily accomplished while the child is laying on their back. In this case, the fasteners are opened, the diaper is removed from under the child, rolled into a roughly cylindrical shape, and then the fasteners are secured around the rolled, soiled diaper, closing the leg openings for hygienic disposal.

In order to avoid having to remove soiled diaper pants from a wearer by sliding the soiled diaper pant down the wearer's legs or tearing bonded side seams, some diaper pants may be configured with tear lines in the front belt or back belt. Such tear lines may include perforations that allow a caregiver to more easily separate the belt along the perforation lines. Once the belt is separated, the diaper pant can be more easily removed from the wearer without having to slide the diaper pant down the wearer's legs, in a similar manner as a traditional open taped diaper form. Once removed from a wearer, a soiled diaper pant may be placed in a disposal configuration by folding or rolling up components of the diaper pant. Some diaper pants may also include disposal fastener tabs or tapes that may enable a caregiver to secure a soiled diaper pant in a folded or rolled up disposal configuration. However, such disposal fastener tabs or tapes may be in locations on the diaper pant that may be undesirably accessible by the wearer.

Consequently, it would be beneficial to create pant-style articles with frangible pathways and disposal fastener components that provide the caregiver the ability to remove and dispose soiled products in a similar manner to traditional open diaper forms. In addition, it would be beneficial to provide diaper pants with disposal fastener components that are hidden or concealed while the diaper pant is worn and/or not readily accessible by a wearer. Further, it would be beneficial to provide diaper pants with frangible pathways configured to help a caregiver successfully determine the location of and access such hidden disposal fastener components.

US20170105883 discloses a pant article comprising a flap joined to a chassis. The flap comprises a first and a second nonwoven and an elastic layer disposed therebetween; and a line of weakness that at least substantially spans from a waist edge to a longitudinally opposite edge. The line of weakness is formed in each of the first and second nonwovens.

US7527617 relates to a disposable wearing article. A tape strip constituting each of the tape fasteners is folded in Z- or inverted Z-shape and attached in such folded state to the outer surface of the wearing article by means of first adhesive.

### SUMMARY OF THE INVENTION

In one form, an absorbent article comprises: a first belt comprising an inner wearer facing surface and an outer garment facing surface, the first belt further comprising a laterally extending inner edge and a laterally extending outer edge, the outer edge positioned longitudinally outward of the inner edge; a second belt, wherein laterally opposing end portions of the second belt are connected with laterally opposing end portions of the first belt at a first side seam and a second side seam to form a waist opening; a chassis comprising a topsheet, a backsheet, and an absorbent core positioned between the topsheet and the backsheet, the chassis comprising a longitudinally extending first side edge and a longitudinally extending second side edge laterally separated from the first side edge by a first end edge and a second end edge longitudinally separated from the first end edge, and wherein longitudinally opposing end regions of the chassis are connected with the first belt and the second belt; wherein a portion of the chassis overlaps the inner wearer facing surface of the first belt to define a chassis overlap region; a frangible pathway in the first belt extending across the overlap region between a proximal terminus on the inner edge and a distal terminus on the outer edge of the first belt, the frangible pathway comprising a first tear zone extending from the overlap region to the distal terminus, and a second tear zone extending from the overlap region to the proximal terminus; and a fastener component on the inner wearer facing surface of the first belt in the overlap region; and an accessibility opening in the first belt in the overlap region positioned between the fastener component and the inner edge of the first belt.

In another form, an absorbent article comprises: a first belt comprising an inner wearer facing surface and an outer garment facing surface, the first belt further comprising a laterally extending inner edge and a laterally extending outer edge, the outer edge positioned longitudinally outward of the inner edge; a second belt, wherein laterally opposing end portions of the second belt are connected with laterally opposing end portions of the first belt at a first side seam and a second side seam to form a waist opening; a chassis comprising a topsheet, a backsheet, and an absorbent core positioned between the topsheet and the backsheet, the chassis comprising a longitudinally extending first side edge and a longitudinally extending second side edge laterally separated from the first side edge by a first end edge and a second end edge longitudinally separated from the first end edge, and wherein longitudinally opposing end regions of the chassis are connected with the first belt and the second belt; wherein a portion of the chassis overlaps the inner wearer facing surface of the first belt to define a chassis overlap region; a fastener component on the inner wearer facing surface of the first belt in the overlap region; and a frangible pathway in the first belt extending across the overlap region between a proximal terminus on the inner edge and a distal terminus on the outer edge of the first belt; wherein the first frangible pathway comprises: a first tear zone extending across the overlap region between the first end edge of the chassis and the fastener component to the distal terminus; and a second tear zone extending across the overlap region between the inner edge of the first belt and the fastener component to the proximal terminus.

In yet another form, an absorbent article comprises: a first belt comprising an inner wearer facing surface and an outer garment facing surface, the first belt further comprising a laterally extending inner edge and a laterally extending outer edge, the outer edge positioned longitudinally outward of the inner edge; a second belt, wherein laterally opposing end portions of the second belt are connected with laterally opposing end portions of the first belt at a first side seam and a second side seam to form a waist opening; a chassis comprising a topsheet, a backsheet, and an absorbent core positioned between the topsheet and the backsheet, the chassis comprising a longitudinally extending first side edge and a longitudinally extending second side edge laterally separated from the first side edge by a first end edge and a second end edge longitudinally separate from the first end edge, and wherein longitudinally opposing end regions of the chassis are connected with the first belt and the second belt; wherein a portion of the chassis overlaps the inner wearer facing surface of the first belt to define a chassis overlap region; a frangible pathway in the first belt extending across the overlap region between a proximal terminus on the inner edge and a distal terminus on the outer edge of the first belt, wherein the first belt is separable along the frangible pathway to define a first belt zone and a second belt zone; and a means for refastenably connecting the first belt zone with at least one other component of the absorbent article in a disposal configuration, the means for refastenably connecting being bonded on the inner wearer facing surface of the first belt in the overlap region; and an accessibility opening in the first belt in the overlap region positioned adjacent the means for refastenably connecting.

In still another form, an absorbent article comprises: a first belt comprising an inner wearer facing surface and an outer garment facing surface, the first belt further comprising a laterally extending inner edge and a laterally extending outer edge, the outer edge positioned longitudinally outward of the inner edge; a second belt, wherein laterally opposing end portions of the second belt are connected with laterally opposing end portions of the first belt at a first side seam and a second side seam to form a waist opening; a chassis comprising a topsheet, a backsheet, and an absorbent core positioned between the topsheet and the backsheet, the chassis comprising a longitudinally extending first side edge and a longitudinally extending second side edge laterally separated from the first side edge by a first end edge and a second end edge longitudinally separated from the first end edge, and wherein longitudinally opposing end regions of the chassis are connected with the first belt and the second belt; wherein a portion of the chassis overlaps the inner wearer facing surface of the first belt to define a chassis overlap region; a frangible pathway in the first belt extending between a proximal terminus on the inner edge and a distal terminus on the outer edge of the first belt; and a fastener component on the inner wearer facing surface of the first belt adapted to connect with at least one other component of the absorbent article in a disposal configuration.

### BRIEF DESCRIPTION OF THE DRAWINGS

Figure 1 shows a perspective view of a diaper pant in a pre-fastened configuration.
Figure 1A shows a perspective view of a diaper pant with a continuous outer cover in a pre-fastened configuration.
Figure 2A shows a plan view of a diaper pant with the portion of the diaper that faces away from a wearer oriented toward the viewer.
Figure 2B shows a plan view of a diaper pant with the portion of the diaper that faces toward a wearer oriented toward the viewer.
Figure 2C shows a plan view of a diaper pant with the portion of the diaper that faces away from a wearer oriented toward the viewer, illustrating first and second belt size and shape features.
Figure 2D shows a plan view of a diaper pant with the portion of the diaper that faces away from a wearer oriented toward the viewer, illustrating first and second belt size and shape features.
Figure 2E shows a plan view of a diaper pant with the portion of the diaper that faces away from a wearer oriented toward the viewer, illustrating first and second belt size and shape features.
Figure 2F shows a plan view of a diaper pant with a continuous outer cover with the portion of the diaper that faces away from a wearer oriented toward the viewer.
Figure 3 is a cross-sectional view of the diaper pant of Figure 2A taken along line 3-3 showing first and second elastic belts provided with panel layers.
Figure 3A is a cross-sectional detailed view of a first belt provided with panel layers wherein one panel layer is folded over another panel layer.

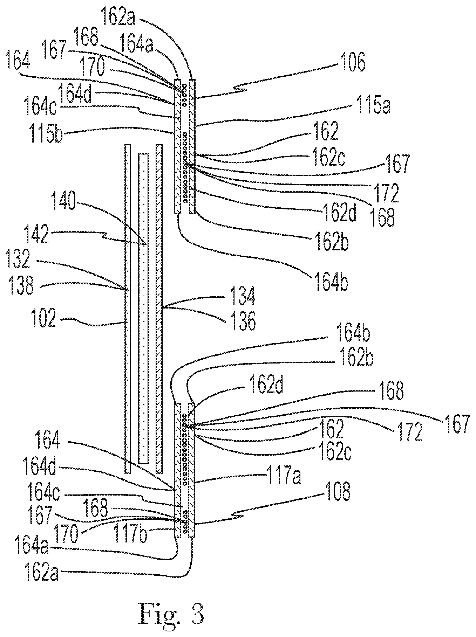

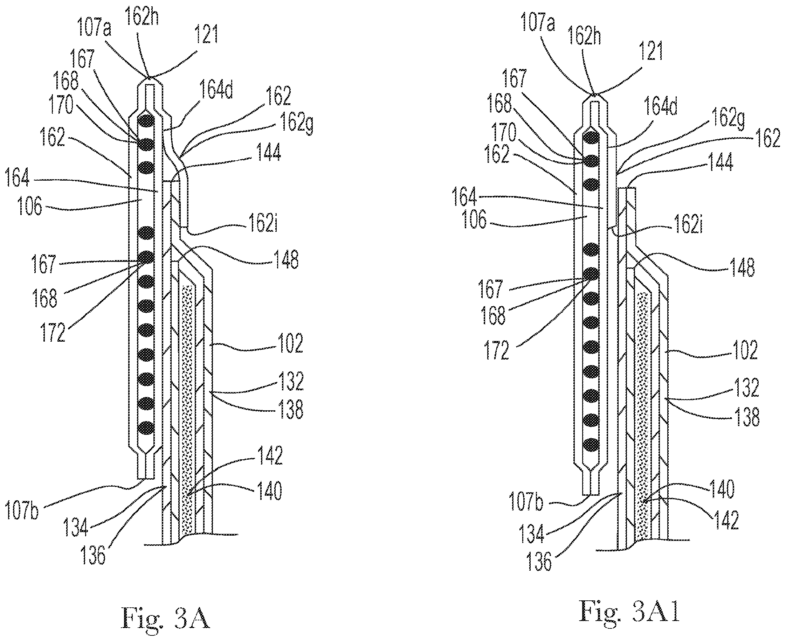

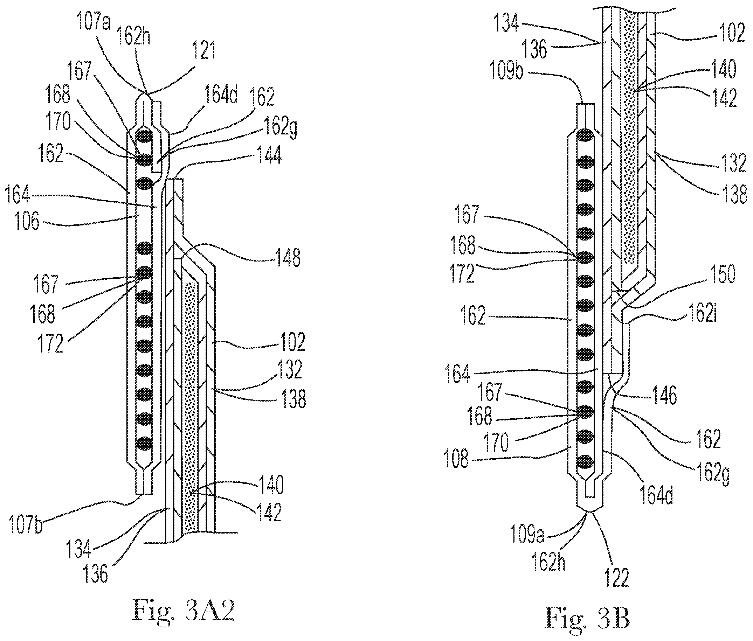

Figure 3A1 is a cross-sectional detailed view of another example configuration wherein the first belt is provided with panel layers wherein one panel layer is folded over another panel layer.
Figure 3A2 is a cross-sectional detailed view of another example configuration wherein the first belt is provided with panel layers wherein one panel layer is folded over another panel layer.
Figure 3B is a cross-sectional detailed view of a second belt provided with panel layers wherein one panel layer is folded over another panel layer.

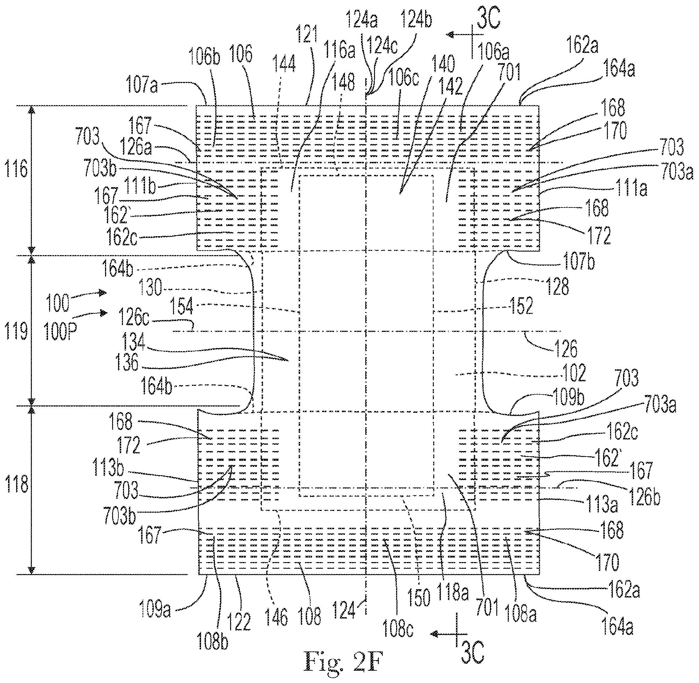

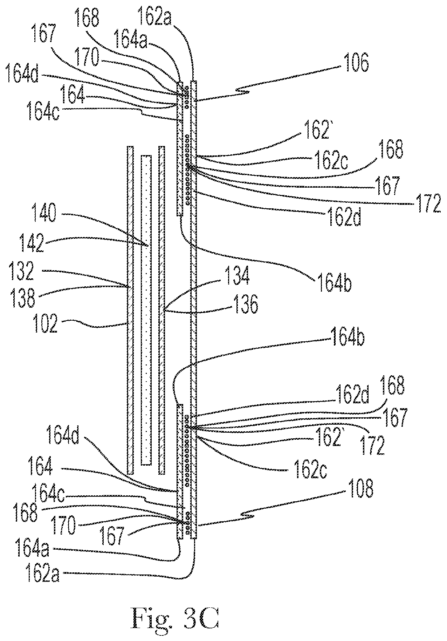

Figure 3C is a cross-sectional view of the diaper pant of Figure 2F taken along line 3C-3C showing first and second elastic belts provided with panel layers and a continuous outer cover.

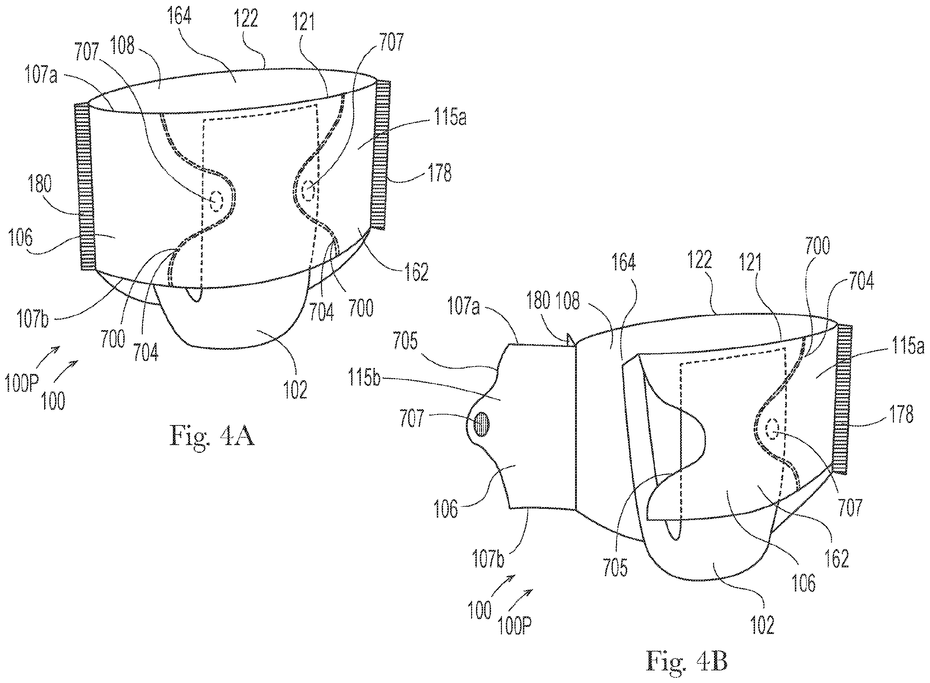

Figure 4A is perspective view of a diaper pant including frangible pathways in a front belt and adjacent an absorbent chassis.
Figure 4B is a perspective view of the diaper pant of Figure 4A showing the front belt having been torn along one of the frangible pathways.

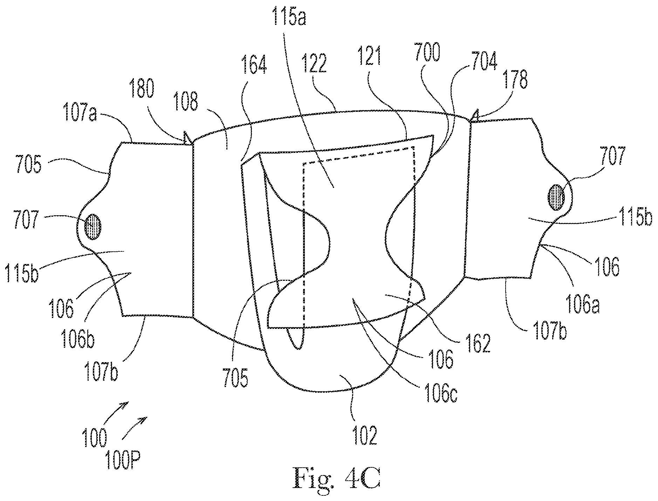

Figure 4C is a perspective view of the diaper pant of Figure 4A showing the front belt having been torn along two frangible pathways.

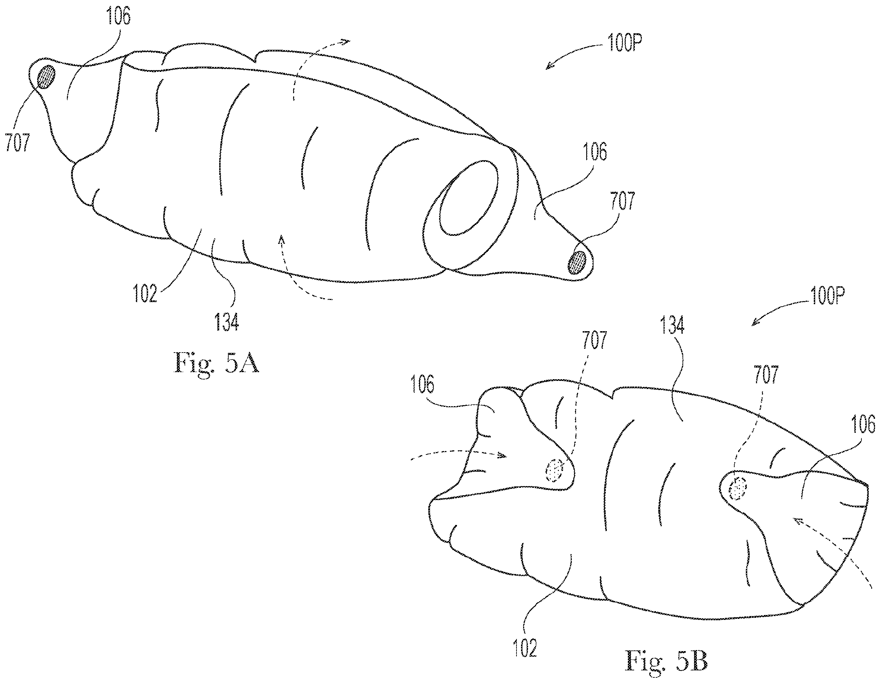

Figure 5A shows the diaper pant of Figure 4C being rolled up onto itself in a longitudinal direction.
Figure 5B shows the diaper pant of Figure 5A with fastener components connected with the backsheet of the chassis to maintain the diaper pant in a disposal configuration.

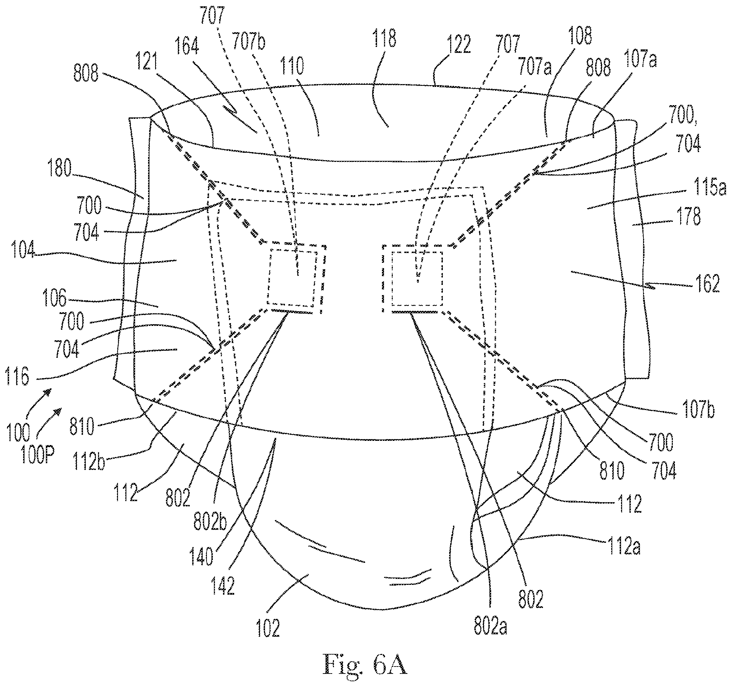

Figure 6A is a perspective view of a diaper pant with frangible pathways.

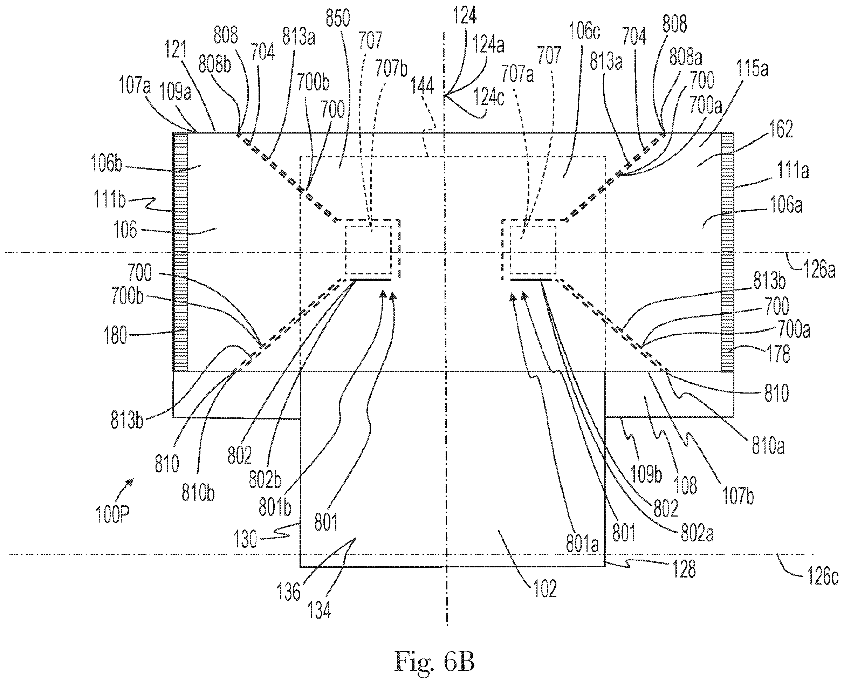

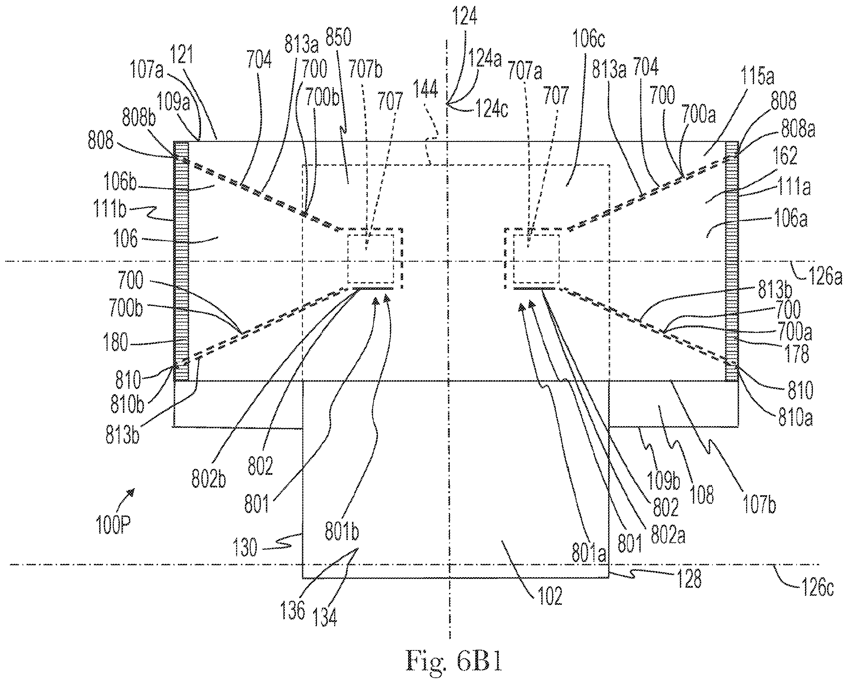

Figure 6B is a front plan view of the diaper pant of Figure 6A.
Figure 6B 1 is a front plan view of another configuration of a diaper pant with frangible pathways having a distal terminus and a proximal terminus positioned on a side seams.

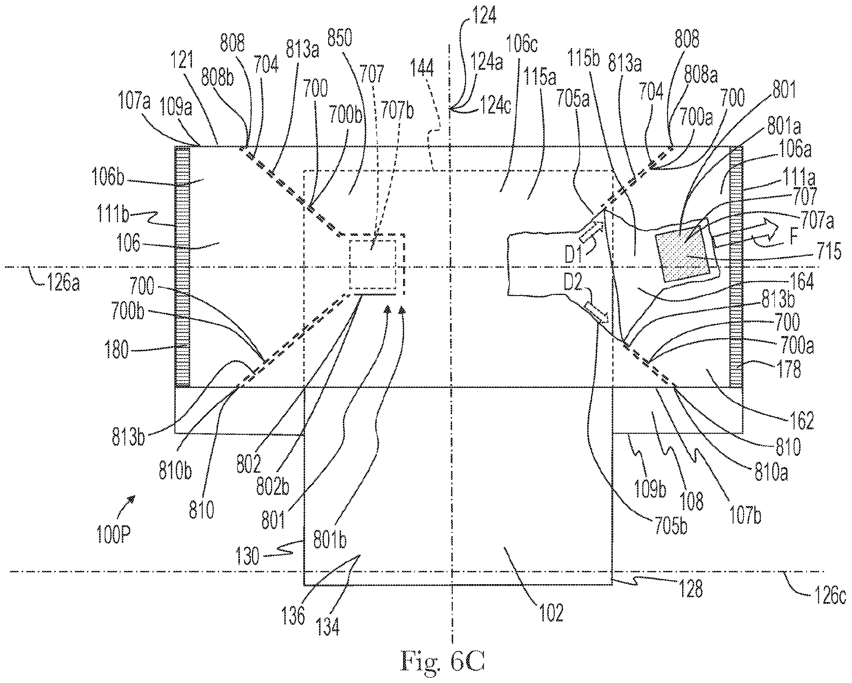

Figure 6C shows a front plan view of the diaper pant of Figure 6B as a first frangible pathway is being torn.

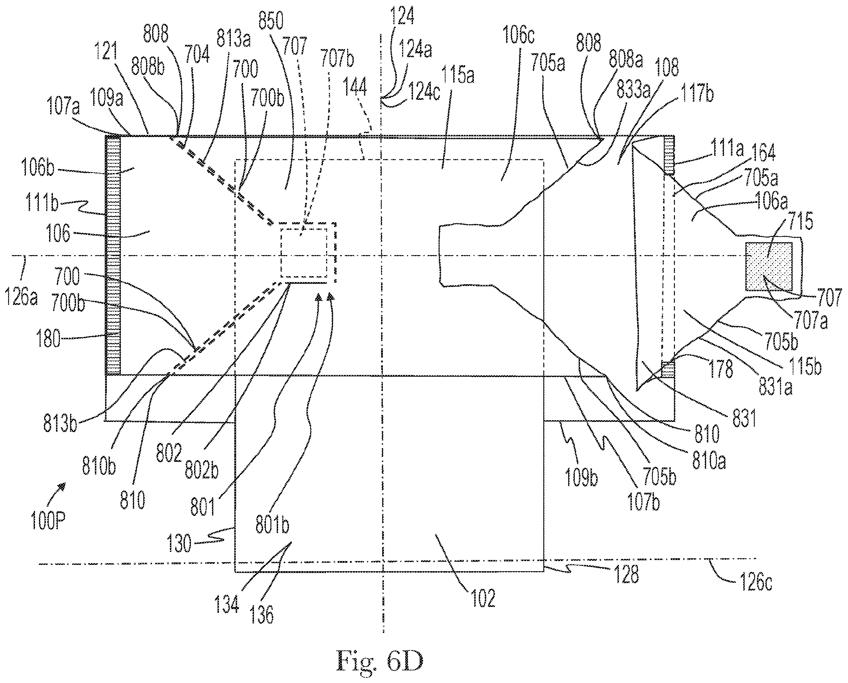

Figure 6D shows a front plan view of the diaper pant of Figure 6C after the first frangible pathway has been completely torn.

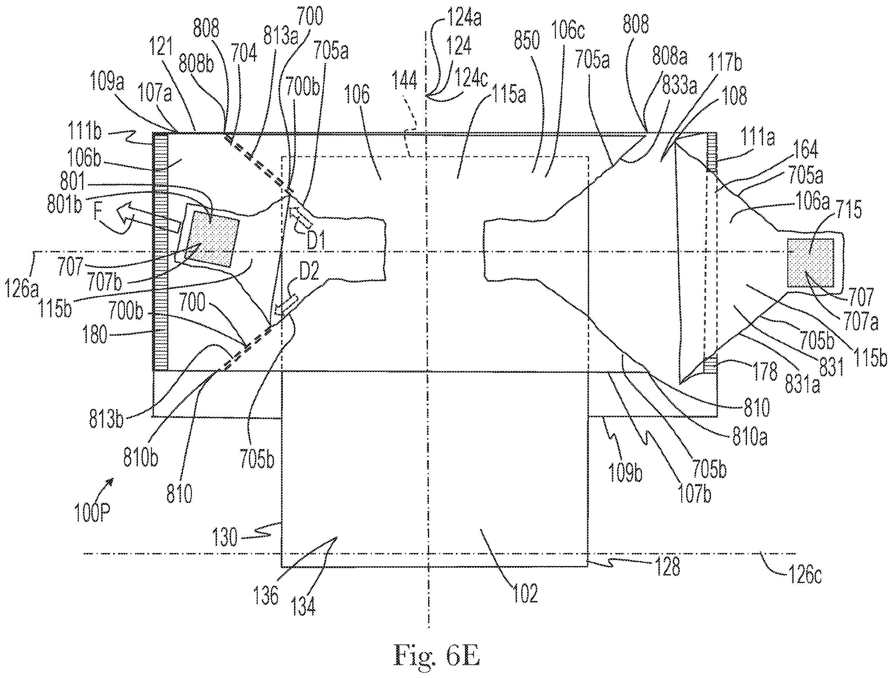

Figure 6E shows a front plan view of the diaper pant of Figure 6D as a second frangible pathway is being torn.

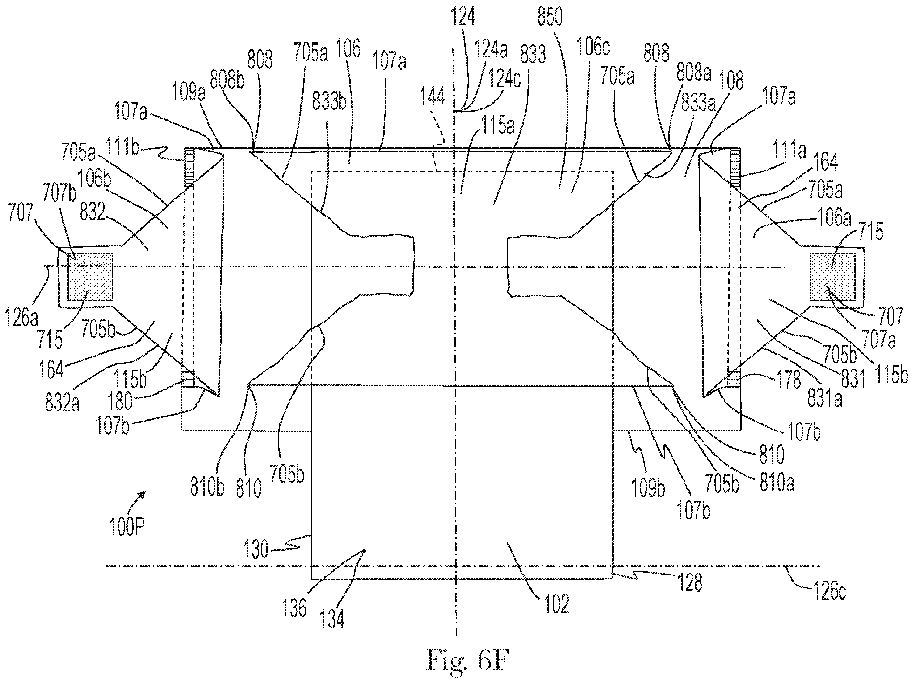

Figure 6F shows a front plan view of the diaper pant of Figure 6E after the second frangible pathway has been completely torn.

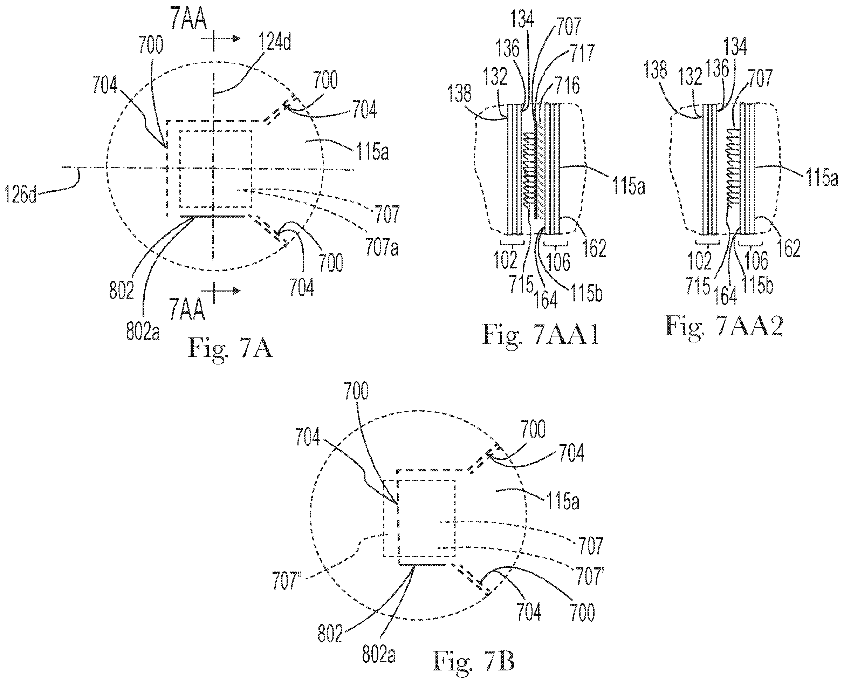

Figure 7A is a detailed view of a fastener component configuration.
Figure 7AA1 is a cross-sectional view of the fastener component of Figure 7A taken along line 7AA-7AA.
Figure 7AA2 is a cross-sectional view of the fastener component of Figure 7A taken along line 7AA-7AA, wherein the fastener component is integrally formed from belt components.
Figure 7B is a detailed view of another fastener component configuration.

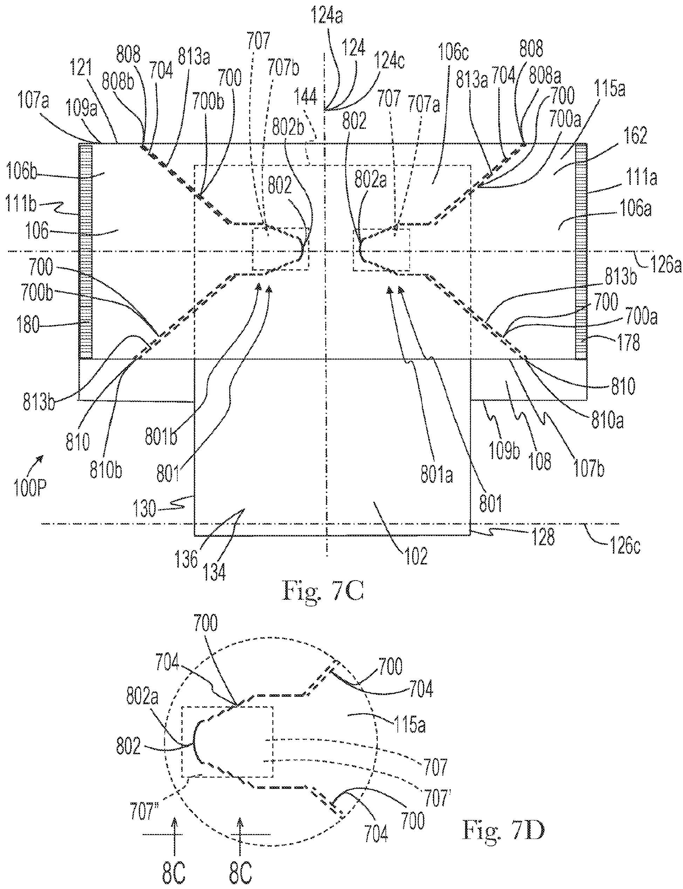

Figure 7C shows a front plan view of a diaper with another configuration of fastener components, accessibility openings, and frangible pathways.
Figure 7D is a detailed view of the fastener component configuration shown in Figure 7C.

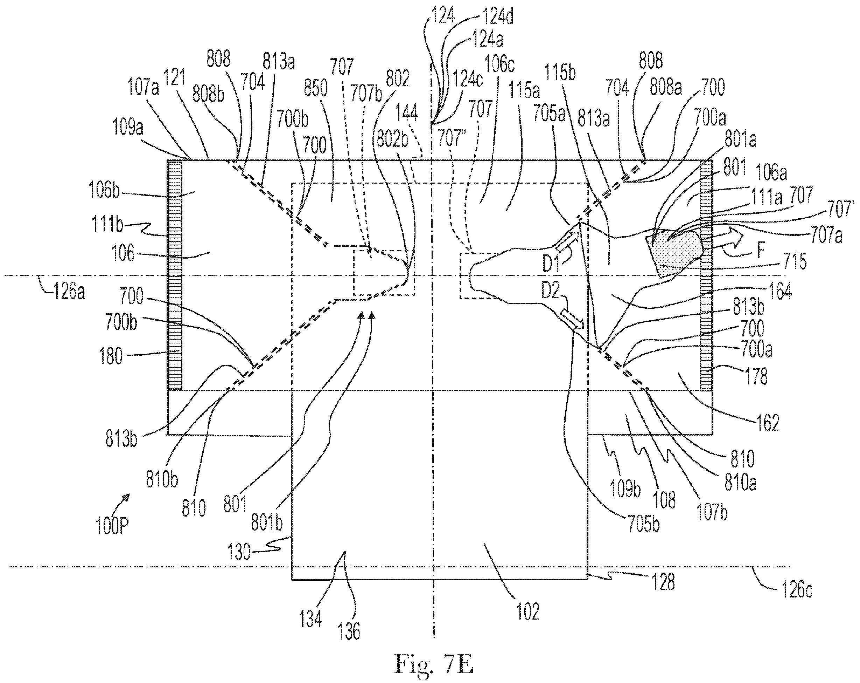

Figure 7E shows a front plan view of the diaper pant of Figure 6B as a first frangible pathway is being torn.

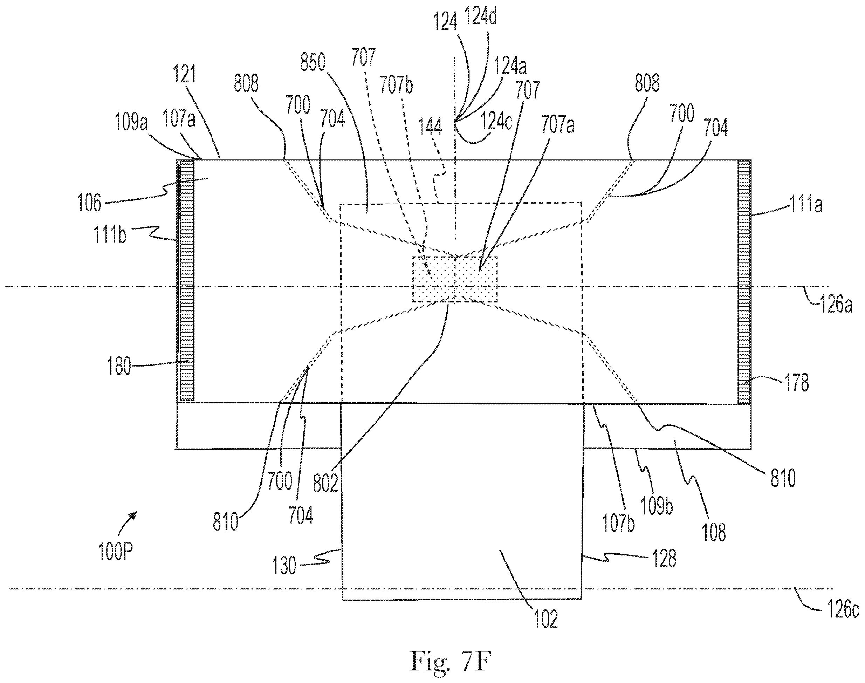

Figure 7F shows a front plan view of a diaper with another configuration of frangible pathways.

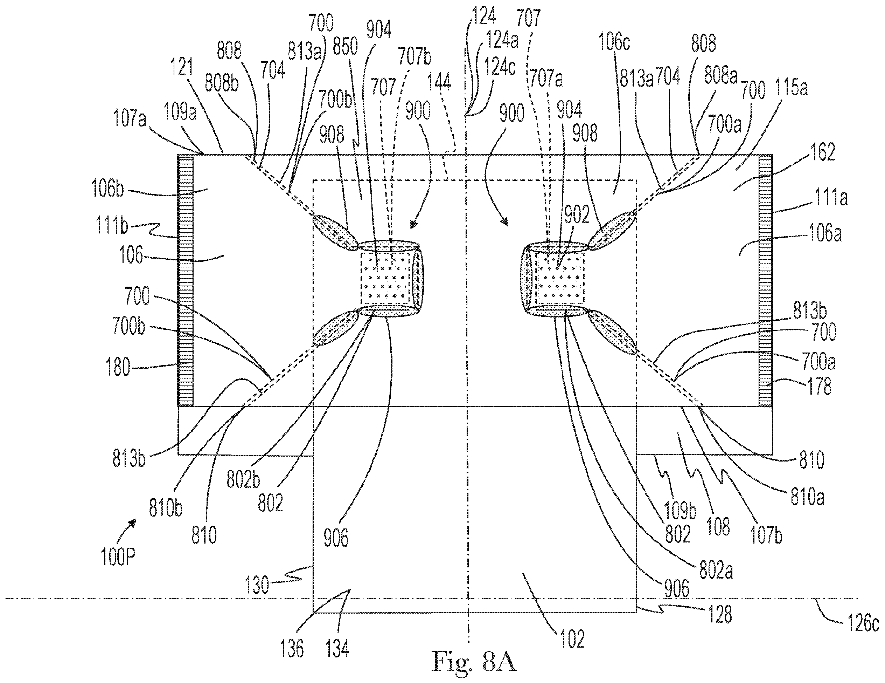

Figure 8A is a front plan view of the diaper pant of Figure 6B showing a generic representation of opening facilitation features.

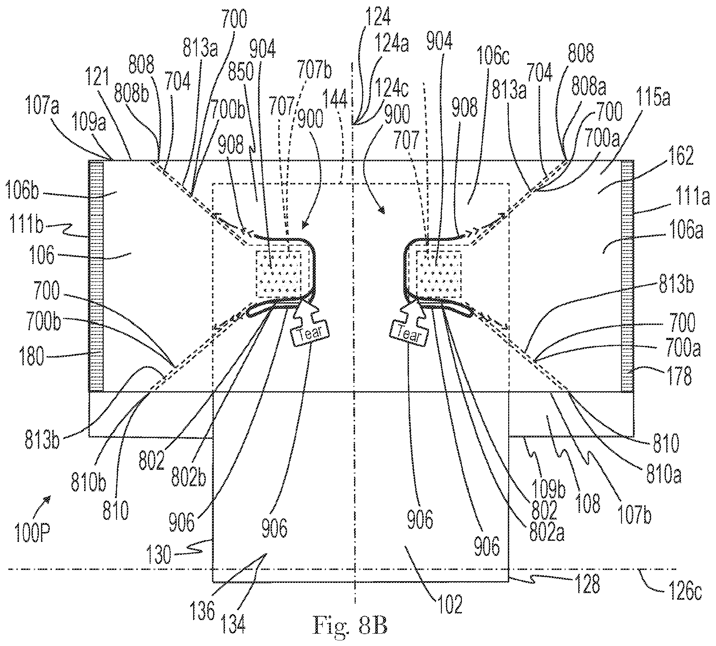

Figure 8B is a front plan view of the diaper pant of Figure 6B showing an example implementation of opening facilitation features.

### DETAILED DESCRIPTION OF THE INVENTION

### DEFINITIONS

The following term explanations may be useful in understanding the present disclosure:
"Absorbent article" refers to devices, which absorb and contain body exudates and, more specifically, refers to devices, which are placed against or in proximity to the body of the wearer to absorb and contain the various exudates discharged from the body. Exemplary absorbent articles include diapers, training pants, pull-on pant-type diapers (i.e., a diaper having a preformed waist opening and leg openings such as illustrated in U.S. Pat. No. 6,120,487), refastenable diapers or pant-type diapers, incontinence briefs and undergarments, diaper holders and liners, feminine hygiene garments such as panty liners, absorbent inserts, menstrual pads and the like.

"Body-facing" and "garment-facing" refer respectively to the relative location of an element or a surface of an element or group of elements. "Body-facing" implies the element or surface is nearer to the wearer during wear than some other element or surface. "Garment-facing" implies the element or surface is more remote from the wearer during wear than some other element or surface (i.e., element or surface is proximate to the wearer's garments that may be worn over the disposable absorbent article).

The terms "elastic," "elastomer" or "elastomeric" refers to materials exhibiting elastic properties, which include any material that upon application of a force to its relaxed, initial length can stretch or elongate to an elongated length more than 10% greater than its initial length and will substantially recover back to about its initial length upon release of the applied force. Elastomeric materials may include elastomeric films, scrims, nonwovens, ribbons, strands and other sheet-like structures.

As used herein, the term "joined" encompasses configurations whereby an element is directly secured to another element by affixing the element directly to the other element, and configurations whereby an element is indirectly secured to another element by affixing the element to intermediate member(s) which in turn are affixed to the other element.

As used herein, the term "distal" is used to describe a position situated away from a center of a body or from a point of attachment, and the term "proximal" is used to describe a position situated nearer to a center of a body or a point of attachment.

The term "substrate" is used herein to describe a material which is primarily two-dimensional (i.e., in an XY plane) and whose thickness (in a Z direction) is relatively small (i.e., 1/10 or less) in comparison to its length (in an X direction) and width (in a Y direction). Non-limiting examples of substrates include a web, layer or layers or fibrous materials, nonwovens, films and foils such as polymeric films or metallic foils. These materials may be used alone or may comprise two or more layers laminated together. As such, a web is a substrate.

The term "nonwoven" refers herein to a material made from continuous (long) filaments (fibers) and/or discontinuous (short) filaments (fibers) by processes such as spunbonding, meltblowing, carding, and the like. Nonwovens do not have a woven or knitted filament pattern.

The term "machine direction" (MD) is used herein to refer to the direction of material flow through a process. In addition, relative placement and movement of material can be described as flowing in the machine direction through a process from upstream in the process to downstream in the process.

The term "cross direction" (CD) is used herein to refer to a direction that is generally perpendicular to the machine direction.

"Pre-strain" refers to the strain imposed on an elastic or elastomeric material prior to combining it with another element of the elastomeric laminate or the absorbent article. Pre-strain is determined by the following equation Pre-strain = ((extended length of the elastic-relaxed length of the elastic)/relaxed length of the elastic)*100.

"Decitex" also known as Dtex is a measurement used in the textile industry used for measuring yarns or filaments. 1 Decitex = 1 gram per 10,000 meters. In other words, if 10,000 linear meters of a yarn or filament weights 500 grams that yarn or filament would have a decitex of 500.

The term "taped diaper" (also referred to as "open diaper") refers to disposable absorbent articles having an initial front waist region and an initial back waist region that are not fastened, pre-fastened, or connected to each other as packaged, prior to being applied to the wearer. A taped diaper may be folded about the lateral centerline with the interior of one waist region in surface to surface contact with the interior of the opposing waist region without fastening or joining the waist regions together. Example taped diapers are disclosed in various suitable configurations U.S. Patent Nos. 5,167,897, 5,360,420, 5,599,335, 5,643,588, 5,674,216, 5,702,551, 5,968,025, 6,107,537, 6,118,041, 6,153,209, 6,410,129, 6,426,444, 6,586,652, 6,627,787, 6,617,016, 6,825,393, and 6,861,571; and U.S. Patent Publication Nos. 2013/0072887 A1; 2013/0211356 A1; and 2013/0306226 A1.

The term "pant" (also referred to as "training pant", "pre-closed diaper", "diaper pant", "pant diaper", and "pull-on diaper") refers herein to disposable absorbent articles having a continuous perimeter waist opening and continuous perimeter leg openings designed for infant or adult wearers. A pant can be configured with a continuous or closed waist opening and at least one continuous, closed, leg opening prior to the article being applied to the wearer. A pant can be preformed or pre-fastened by various techniques including, but not limited to, joining together portions of the article using any refastenable and/or permanent closure member (e.g., seams, heat bonds, pressure welds, adhesives, cohesive bonds, mechanical fasteners, etc.). A pant can be preformed anywhere along the circumference of the article in the waist region (e.g., side fastened or seamed, front waist fastened or seamed, back waist fastened or seamed). Example diaper pants in various configurations are disclosed in U.S. Patent Nos. 4,940,464; 5,092,861; 5,246,433; 5,569,234; 5,897,545; 5,957,908; 6,120,487; 6,120,489; 7,569,039 and U.S. Patent Publication Nos. 2003/0233082 A1; 2005/0107764 A1, 2012/0061016 A1, 2012/0061015 A1; 2013/0255861 A1; 2013/0255862 A1; 2013/0255863 A1; 2013/0255864 A1; and 2013/0255865 A1.

"Closed-form" means opposing waist regions are joined, as packaged, either permanently or refastenably to form a continuous waist opening and leg openings.

"Open-form" means opposing waist regions are not initially joined to form a continuous waist opening and leg openings but comprise a closure means such as a fastening system to join the waist regions to form the waist and leg openings before or during application to a wearer of the article.

The present disclosure relates to absorbent articles having front and/or back waist regions including one or more frangible pathways with concealed disposal fastener components. In some configurations, an absorbent article may comprise a first belt and a second belt, each belt comprising a first end region and a second end region laterally separated from the first end region by a central region. The first end region of the first belt is connected with the first end region of the second belt at a first side seam and the second end region of the first belt is connected with the second end region of the second belt at a second side seam to form a waist opening. The absorbent article may further comprise a chassis comprising a topsheet, a backsheet, and an absorbent core positioned between the topsheet and the backsheet. The chassis may further comprise a first end region and a second end region longitudinally separated from the first end region by a crotch region. Longitudinally opposing end regions of the chassis may be connected with the first belt and the second belt, and a portion of the chassis that overlaps an inner wearer facing surface of the first belt may define a chassis overlap region.

As discussed in more detail below, the first belt and/or the second belt may comprise one or more frangible pathways. For example, a frangible pathway in the first and/or second belt may extend across the overlap region between a proximal terminus on the inner edge and a distal terminus on the outer edge of the first belt and/or the second belt. The frangible pathway may comprise a first tear zone extending from the overlap region to the distal terminus, and a second tear zone extending from the overlap region to the proximal terminus. A fastener component may be positioned on the inner wearer facing surface of the first belt and/or second belt in the overlap region, and an accessibility opening in the first belt and/or the second belt in the overlap region may be positioned between the fastener component and the inner edge of the first belt and/or the second belt. As discussed in more detail below, the accessibility opening may be positioned adjacent an edge of the fastener component and/or may be positioned within the fastener component. Such frangible pathway configurations provide a feature that allows an elastic belt of a diaper pant to be relatively easily torn along the frangible pathway when removing the diaper pant from a wearer, and the fastener component may provide a feature that helps maintain a used diaper pant in a disposal configuration. For example, the first belt may be separable along the frangible pathway to define a first belt zone and a second belt zone, wherein the first belt zone is laterally between the first side seam and the second belt zone, and wherein the fastener component is adapted to refastenably connect the first belt zone with at least one other component of the absorbent article in a disposal configuration.

Positioning fastener components on the inner wearing facing surface of the first belt and/or the second belt helps to conceal the fastener component from the wearer while wearing the diaper pant. In addition, positioning the accessibility opening adjacent the fastener component may help a caregiver successfully determine the location of and access such hidden disposal fastener components. Further, an absorbent article may also comprise one or more opening facilitation features, such as opening operational indicia and opening mechanical aids to help a user to successfully determine the location and/or function of the fastener component.

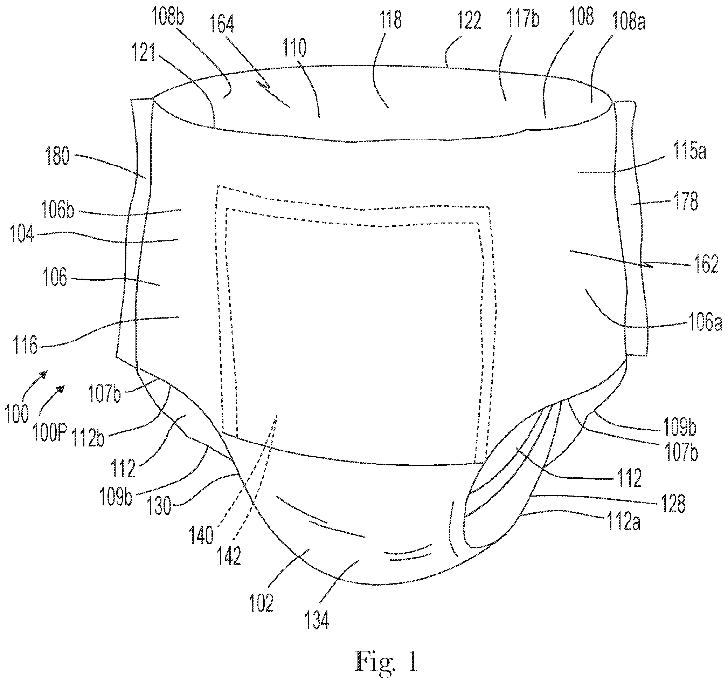

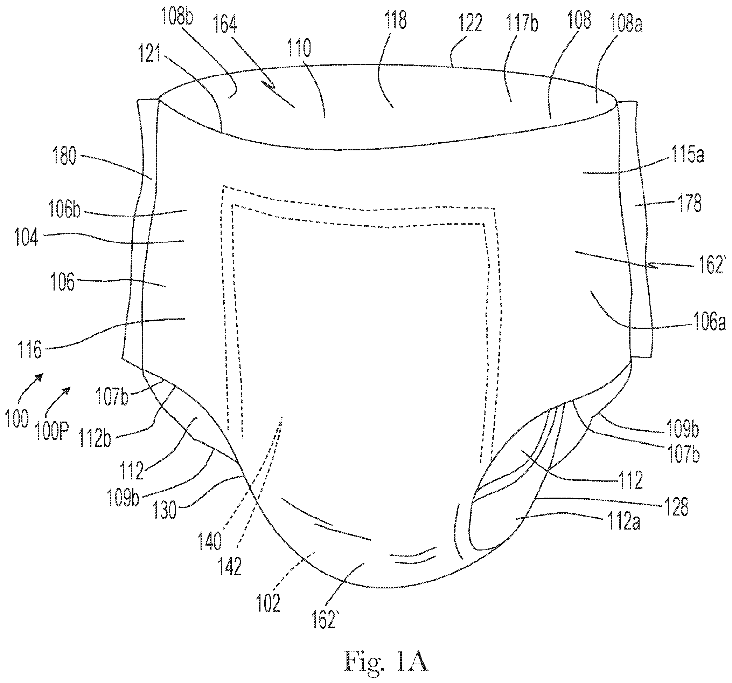

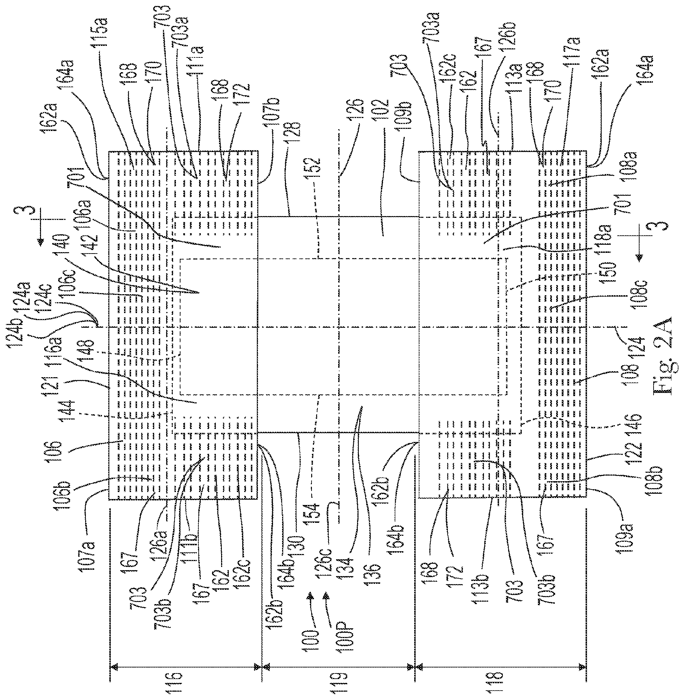

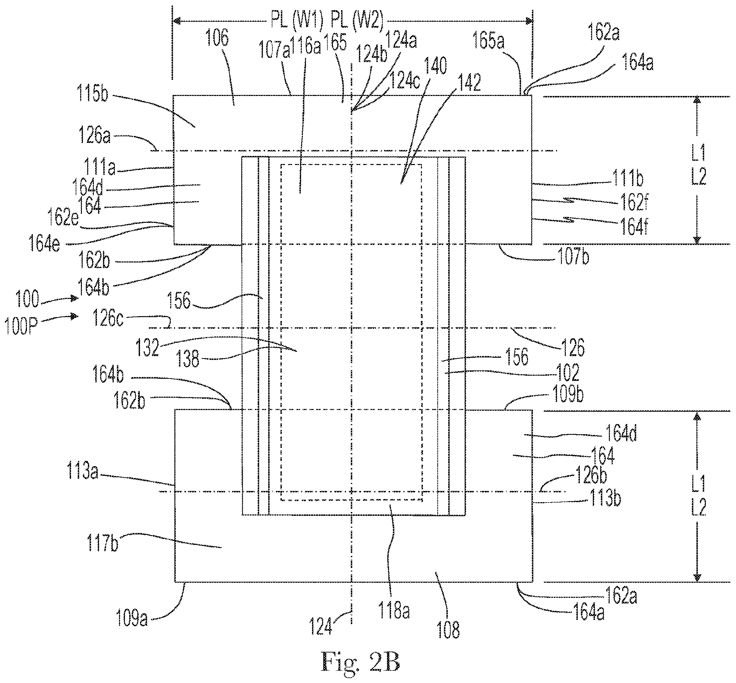

Figures 1-2B show an example of an absorbent article 100 in the form of a diaper pant 100P that may include components constructed in accordance with the configurations disclosed herein. In particular, Figure 1 shows a perspective views of a diaper pant 100P in a pre-fastened configuration. Figure 2A shows a plan view of the diaper pant 100P with the portion of the diaper that faces away from a wearer oriented toward the viewer, and Figure 2B shows a plan view of the diaper pant 100P with the portion of the diaper that faces toward a wearer oriented toward the viewer. The diaper pant 100P includes a chassis 102 and a ring-like elastic belt 104. As discussed below in more detail, a first elastic belt 106 and a second elastic belt 108 are bonded together to form the ring-like elastic belt 104.

With continued reference to Figures 1-2B, the diaper pant 100P and the chassis 102 each include a first waist region 116, a second waist region 118, and a crotch region 119 disposed intermediate the first and second waist regions. It may also be described that the chassis 102 includes a first end region 116a, a second end region 118a, and a crotch region 119 disposed intermediate the first and second end regions 116a, 118a. The first waist region 116 may be configured as a front waist region, and the second waist region 118 may be configured as back waist region. The diaper 100P may also include a laterally extending front waist edge 121 in the front waist region 116 and a longitudinally opposing and laterally extending back waist edge 122 in the back waist region 118. To provide a frame of reference for the present discussion, the diaper 100P and chassis 102 of Figures 2A and 2B are shown with a longitudinal axis 124 and a lateral axis 126. In some embodiments, the longitudinal axis 124 may extend through the front waist edge 121 and through the back waist edge 122. And the lateral axis 126 may extend through a first longitudinal or right side edge 128 and through a second longitudinal or left side edge 130 of the chassis 102. As previously mentioned, the longitudinal axis 124 extends perpendicularly through the front waist edge 121 and the back waist edge 122, and the lateral axis 126 extends perpendicularly to the longitudinal axis 124. When the diaper pant 100P is worn, the longitudinal direction may extend from the wearer's front waist, through the crotch, to the wearer's back waist. To provide a further frame of reference for the present discussion, the diapers 100P of Figures 2A, 2B, and 18B are shown wherein: the first elastic belt 106 comprises a longitudinal centerline 124a and lateral centerline 126a; the second elastic belt 108 comprises a longitudinal centerline 124b and lateral centerline 126b; and the chassis 102 comprises a longitudinal centerline 124c and lateral centerline 126c. The longitudinal centerlines 124a, 124b, 124c are perpendicular to the lateral center lines 126a, 126b, 126c.

As shown in Figures 1-2B, the diaper pant 100P may include an inner, body facing surface 132, and an outer, garment facing surface 134. The chassis 102 may include a backsheet 136 and a topsheet 138. The chassis 102 may also include an absorbent assembly 140, including an absorbent core 142, disposed between a portion of the topsheet 138 and the backsheet 136. As discussed in more detail below, the diaper 100P may also include other features, such as leg elastics and/or leg cuffs to enhance the fit around the legs of the wearer.

As shown in Figure 2A, the periphery of the chassis 102 may be defined by the first longitudinal side edge 128, a second longitudinal side edge 130, a first laterally extending end edge 144 disposed in the first waist region 116, and a second laterally extending end edge 146 disposed in the second waist region 118. Both side edges 128 and 130 extend longitudinally between the first end edge 144 and the second end edge 146. As shown in Figure 2A, the laterally extending end edges 144 and 146 may be located longitudinally inward from the laterally extending front waist edge 121 in the front waist region 116 and the laterally extending back waist edge 122 in the back waist region 118. In some configurations, the laterally extending end edges 144 and 146 may be coterminous with or located longitudinally outward from the laterally extending front waist edge 121 in the front waist region 116 and the laterally extending back waist edge 122 in the back waist region 118. When the diaper pant 100P is worn on the lower torso of a wearer, the front waist edge 121 and the back waist edge 122 may encircle a portion of the waist of the wearer. At the same time, the side edges 128 and 130 may encircle at least a portion of the legs of the wearer. And the crotch region 119 may be generally positioned between the legs of the wearer with the absorbent core 142 extending from the front waist region 116 through the crotch region 119 to the back waist region 118.

As previously mentioned, the diaper pant 100P may include a backsheet 136. The backsheet 136 may also define the outer, garment facing surface 134 of the chassis 102. The backsheet 136 may also comprise a woven or nonwoven material, polymeric films such as thermoplastic films of polyethylene or polypropylene, and/or a multi-layer or composite materials comprising a film and a nonwoven material. The backsheet may also comprise an elastomeric film. An example backsheet 136 may be a polyethylene film having a thickness of from about 0.012 mm (0.5 mils) to about 0.051 mm (2.0 mils). Further, the backsheet 136 may permit vapors to escape from the absorbent core (i.e., the backsheet is breathable) while still preventing exudates from passing through the backsheet 136.

Also described above, the diaper pant 100P may include a topsheet 138. The topsheet 138 may also define all or part of the inner, wearer facing surface 132 of the chassis 102. The topsheet 138 may be liquid pervious, permitting liquids (e.g., menses, urine, and/or runny feces) to penetrate through its thickness. A topsheet 138 may be manufactured from a wide range of materials such as woven and nonwoven materials; apertured or hydroformed thermoplastic films; apertured nonwovens, porous foams; reticulated foams; reticulated thermoplastic films; and thermoplastic scrims. Woven and nonwoven materials may comprise natural fibers such as wood or cotton fibers; synthetic fibers such as polyester, polypropylene, or polyethylene fibers; or combinations thereof. If the topsheet 138 includes fibers, the fibers may be spunbond, carded, wet-laid, meltblown, hydroentangled, or otherwise processed as is known in the art. Topsheets 138 may be selected from high loft nonwoven topsheets, apertured film topsheets and apertured nonwoven topsheets. Exemplary apertured films may include those described in U.S. Patent Nos. 5,628,097; 5,916,661; 6,545,197; and 6,107,539.

As mentioned above, the diaper pant 100P may also include an absorbent assembly 140 that is joined to the chassis 102. As shown in Figure 2A, the absorbent assembly 140 may have a laterally extending front edge 148 in the front waist region 116 and may have a longitudinally opposing and laterally extending back edge 150 in the back waist region 118. The absorbent assembly may have a longitudinally extending right side edge 152 and may have a laterally opposing and longitudinally extending left side edge 154, both absorbent assembly side edges 152 and 154 may extend longitudinally between the front edge 148 and the back edge 150. The absorbent assembly 140 may additionally include one or more absorbent cores 142 or absorbent core layers. The absorbent core 142 may be at least partially disposed between the topsheet 138 and the backsheet 136 and may be formed in various sizes and shapes that are compatible with the diaper. Exemplary absorbent structures for use as the absorbent core of the present disclosure are described in U.S. Patent Nos. 4,610,678; 4,673,402; 4,888,231; and 4,834,735.

Some absorbent core embodiments may comprise fluid storage cores that contain reduced amounts of cellulosic airfelt material. For instance, such cores may comprise less than about 40%, 30%, 20%, 10%, 5%, or even 1% of cellulosic airfelt material. Such a core may comprise primarily absorbent gelling material in amounts of at least about 60%, 70%, 80%, 85%, 90%, 95%, or even about 100%, where the remainder of the core comprises a microfiber glue (if applicable). Such cores, microfiber glues, and absorbent gelling materials are described in U.S. Patent Nos. 5,599,335; 5,562,646; 5,669,894; and 6,790,798 as well as U.S. Patent Publication Nos. 2004/0158212 A1 and 2004/0097895 A1.

As previously mentioned, the diaper 100P may also include elasticized leg cuffs 156. It is to be appreciated that the leg cuffs 156 can be and are sometimes also referred to as leg bands, side flaps, barrier cuffs, elastic cuffs or gasketing cuffs. The elasticized leg cuffs 156 may be configured in various ways to help reduce the leakage of body exudates in the leg regions. Example leg cuffs 156 may include those described in U.S. Patent Nos. 3,860,003; 4,909,803; 4,695,278; 4,795,454; 4,704,115; 4,909,803; and U.S. Patent Publication No. 2009/0312730 A1.

As mentioned above, diaper pants may be manufactured with a ring-like elastic belt 104 and provided to consumers in a configuration wherein the front waist region 116 and the back waist region 118 are connected to each other as packaged, prior to being applied to the wearer. As such, diaper pants may have a continuous perimeter waist opening 110 and continuous perimeter leg openings 112 such as shown in Figure 1. The ring-like elastic belt may be formed by joining a first elastic belt to a second elastic belt with a permanent side seam or with an openable and reclosable fastening system disposed at or adjacent the laterally opposing sides of the belts.

As previously mentioned, the ring-like elastic belt 104 may be defined by a first elastic belt 106 connected with a second elastic belt 108. As shown in Figures 2A and 2B, the first elastic belt 106 extends between a first longitudinal side edge 111a and a second longitudinal side edge 111b and defines first and second opposing end regions 106a, 106b and a central region 106c. And the second elastic 108 belt extends between a first longitudinal side edge 113a and a second longitudinal side edge 113b and defines first and second opposing end regions 108a, 108b and a central region 108c. As measured in an extended state, the distance between the first longitudinal side edge 111a and the second longitudinal side edge 111b defines the pitch length, PL, of the first elastic belt 106, and the distance between the first longitudinal side edge 113a and the second longitudinal side edge 113b defines the pitch length, PL, of the second elastic belt 108. The central region 106c of the first elastic belt is connected with the first waist region 116 or first end region 116a of the chassis 102, and the central region 108c of the second elastic belt 108 is connected with the second waist region 118 or second end region 118a of the chassis 102. As shown in Figure 1, the first end region 106a of the first elastic belt 106 is connected with the first end region 108a of the second elastic belt 108 at first side seam 178, and the second end region 106b of the first elastic belt 106 is connected with the second end region 108b of the second elastic belt 108 at second side seam 180 to define the ring-like elastic belt 104 as well as the waist opening 110 and leg openings 112. It is to be appreciated that the first belt 106 and the second belt 108 may be permanently or refastenably connected with each other at the first side seam 178 and the second side seam 180. The side seams 178, 180 may comprise a permanent bond, such as a thermal, pressure, or adhesive bond, or may be a releasable bond, such as a mechanical or cohesive fastener.

As shown in Figures 2A and 2B, the first elastic belt 106 also defines an outer laterally extending edge 107a and an inner laterally extending edge 107b, and the second elastic belt 108 defines an outer laterally extending edge 109a and an inner laterally extending edge 109b. The outer edge 107a of the first belt 106 is positioned longitudinally outward of the inner edge 107b, and the outer edge 109a of the second belt 108 is positioned longitudinally outward of the inner edge 109b. As such, as shown in Figure 1, a perimeter edge 112a of one leg opening may be defined by portions of the inner laterally extending edge 107b of the first elastic belt 106, the inner laterally extending edge 109b of the second elastic belt 108, and the first longitudinal or right side edge 128 of the chassis 102. And a perimeter edge 112b of the other leg opening may be defined by portions of the inner laterally extending edge 107b, the inner laterally extending edge 109b, and the second longitudinal or left side edge 130 of the chassis 102. The outer laterally extending edges 107a, 109a may also define the front waist edge 121 and the laterally extending back waist edge 122 of the diaper pant 100P.

It is to be appreciated that the first elastic belt 106 and the second elastic belt 108 may define different sizes and shapes. In some configurations, the first elastic belt 106 and/or second elastic belt 108 may define curved contours. For example, the inner lateral edges 107b, 109b of the first and/or second elastic belts 106, 108 may include non-linear or curved portions in the first and second opposing end regions. Such curved contours may help define desired shapes to leg opening 112, such as for example, relatively rounded leg openings. In addition to having curved contours, the elastic belts 106, 108 may include elastic strands 168 that extend along non-linear or curved paths that may correspond with the curved contours of the inner lateral edges 107b, 109b.

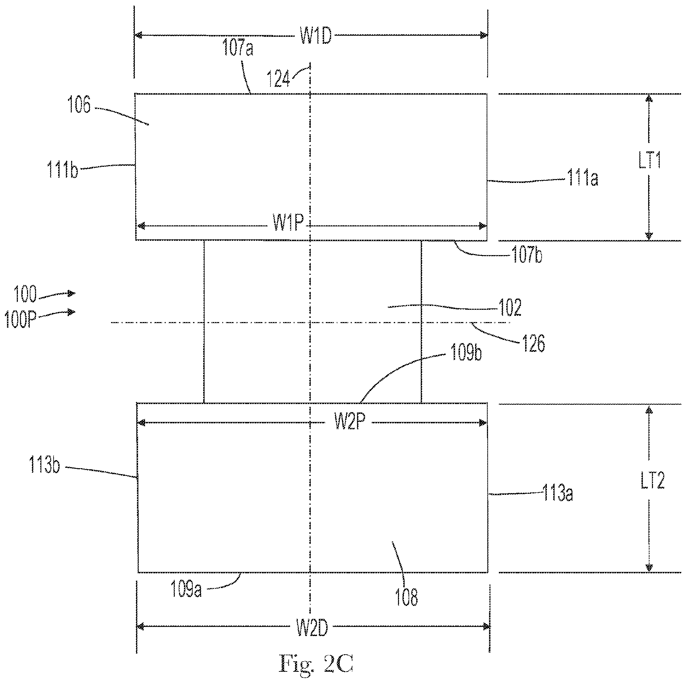

Figure 2C shows a configuration wherein the first elastic belt 106 and the second elastic belt 108 both define generally rectangular shapes. For example, as shown in Figure 2C, the outer laterally extending edge 107a of the first elastic belt 106 may comprise a lateral width of W1D and the inner laterally extending edge 107b may comprise a lateral width of W1P, wherein W1D and W1P are equal or substantially equal. In addition, the outer laterally extending edge 109a of the second elastic belt 108 may comprise a lateral width of W2D and the inner laterally extending edge 109b may comprise a lateral width of W2P, wherein W2D and W2P are equal or substantially equal.

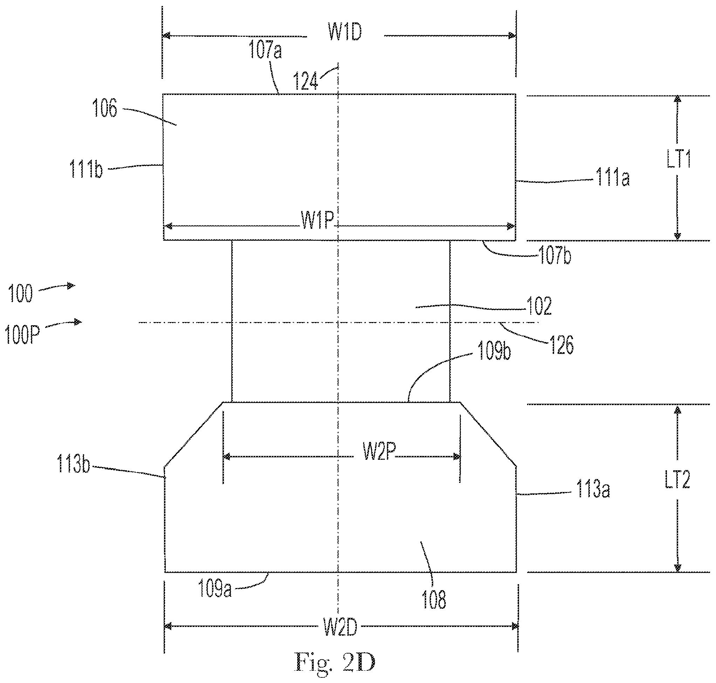

In some configurations, at least one of the first elastic belt 106 and the second elastic belt 108 may comprise lateral edges having different lengths. For example, Figure 2D shows a configuration wherein the first elastic belt 106 defines a generally rectangular shape, such as described with reference to Figure 2C, and wherein the outer laterally extending edge 109a of the second elastic belt 108 and the inner laterally extending edge 109b have different lengths. As shown in Figure 2D, the outer laterally extending edge 109a of the second elastic belt 108 may comprise a lateral width of W2D and the inner laterally extending edge 109b may comprise a lateral width of W2P, wherein W2D is greater than W2P.

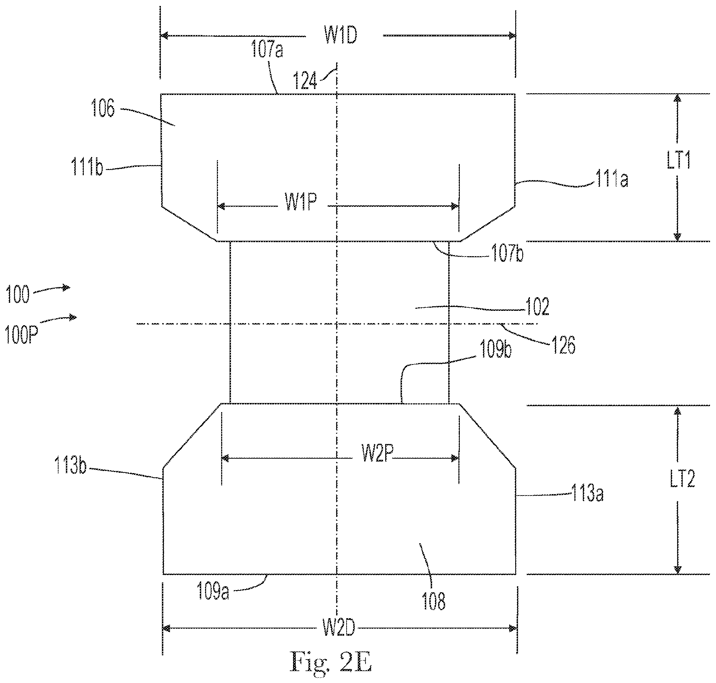

In some configurations, both the first elastic belt 106 and the second elastic belt 108 may comprise lateral edges having different lengths. For example, Figure 2E shows a configuration wherein the outer laterally extending edge 107a of the first elastic belt 106 and the inner laterally extending edge 107b have different lengths, and wherein the outer laterally extending edge 109a of the second elastic belt 108 and the inner laterally extending edge 109b have different lengths. As shown in Figure 2E, the outer laterally extending edge 107a of the first elastic belt 107 may comprise a lateral width of W1D and the inner laterally extending edge 107b may comprise a lateral width of W1P, wherein W1D is greater than W1P, and wherein the outer laterally extending edge 109a of the second elastic belt 108 may comprise a lateral width of W2D and the inner laterally extending edge 109b may comprise a lateral width of W2P, wherein W2D is greater than W2P.

With reference to Figures 2C-2E, the first elastic belt 106 may define a longitudinal length LT1 extending between outer laterally extending edge 107a and the inner laterally extending edge 107b, and the second elastic belt 108 may define a longitudinal length LT2 extending between outer laterally extending edge 109a and the inner laterally extending edge 109b. In some configurations, LT1 may be equal to LT2. In some configurations, LT1 may be less or greater than LT2. With continued reference to Figures 2C-2E, in some configurations, W1D may be equal to W1P, or W1D may be different than W1P. In some configurations, W2D may be equal to W2P, or W2D may be different than W2P. In some configurations, W1D and/or W1P may be equal to or different W2D and/or W2P.

With reference to Figures 2A, 2B, and 3, the first elastic belt 106 and the second elastic belt 108 may also each include a first substrate 162 and a second substrate 164. The first substrates 162 may be oriented to define at least a portion of a garment facing surface 115a of the first elastic belt 106 and a garment facing surface 117a the second elastic belt 108, and the second substrates 164 may be oriented to define at least a portion of a wearer facing surface 115b of the first elastic belt 106 and a wearer facing surface 117b of the second elastic belt 108. The first substrate 162 may extend from a proximal edge 162b to a distal edge 162a for a maximum length L1, and the second substrate 164 may extend from a proximal edge 164b to a distal edge 164a for a maximum length L2. It is to be appreciated that the distal edge 162a and/or the proximal edge 162b of the first substrate 162 may be straight and/or curved and/or may be parallel or unparallel to each other. It is also to be appreciated that the distal edge 164a and/or the proximal edge 164b of the second substrate 164 may be straight and/or curved and/or may be parallel or unparallel to each other. As such, the maximum length L1 refers to the longest distance extending longitudinally between the distal edge 162a and the proximal edge 162b of the first substrate 162, and the maximum length L2 refers to the longest distance extending longitudinally between the distal edge 164a and the proximal edge 164b of the second substrate 164. In some configurations, L1 may be equal to, less than, or greater than L2. In some configurations, L1 may be equal to or less than LT1, and L2 may be equal to or less than LT2. In some configurations, the distal edge 162a of the first substrate 162 may define at least a portion of the front waist edge 121 and/or at least a portion of back waist edge 122, and/or the distal edge 164a of the second substrate 164 may define at least a portion of the front waist edge 121 and/or at least a portion of back waist edge 122. As such, in some configurations, the distal edge 162a of the first substrate 162 and/or the distal edge 164a of the second substrate 164 may define at least a portion of the waist opening 110.

It is also to be appreciated that the first substrate 162 and/or the second substrate 164 may extend continuously from the first belt 106 to the second belt 108. For example, the first substrate 162 may be configured to define a continuous outer cover 162' that extends contiguously from the first waist edge 121 to the second waist edge 122, such as shown in Figures 1A, 2F, and 3C. It is also to be appreciated that diaper pants 100P with continuous outer covers, such as shown in Figures 1A, 2F, and 3C may also be configured to include various aspects of the frangible pathways and fastener components discussed herein.

It is to be appreciated that the first substrate 162 and the second substrate 164 may define various lateral widths that may or may not be equal. For example, as shown in Figure 2B, the first substrate 162 may extend laterally between a first longitudinal edge 162e and a second longitudinal edge 162f to define a first lateral width W1, and the second substrate 164 may extend laterally between a first longitudinal edge 164e and a second longitudinal edge 164f to define a second lateral width W2.

In some configurations, the proximal edge 162b of the first substrate 162 and/or the proximal edge 164b of the second substrate 164 may extend laterally across the backsheet 136. As shown in Figures 2A-3, the first substrate 162 includes a garment facing surface 162c and an opposing wearer facing surface 162d, and the second substrate 164 includes a garment facing surface 164c and an opposing wearer facing surface 164d.

In some configurations, the first elastic belt 106 and/or the second elastic belt 108 may include a folded portion of at least the first substrate 162 and/or the second substrate 164. For example, as shown in Figures 3A and 3B, the first elastic belt 106 and/or the second elastic belt 108 may include a folded portion 162g of the first substrate 162 extending longitudinally between a fold line 162h in the first substrate 162 and a lateral edge 162i. As such, the folded portion 162g of the first substrate 162 may be connected with the wearer facing surface 164d of the second substrate 164. In some configurations, the folded portion 162g of the first substrate 162 may also be connected with and/or overlap the chassis 102. In some configurations, the folded portion 162g of the first substrate 162 may also be connected with the wearer facing surface 162d of the first substrate 162. In some configurations, a portion of the folded portion 162g of the first substrate 162 may be left unbonded to the chassis 102 and/or the second substrate 164, forming a pocket having an opening oriented toward the lateral centerline 162c of the chassis 102. In another example, the first elastic belt 106 and/or the second elastic belt 108 may include a folded portion of the second substrate 164 extending longitudinally between a fold line in the second substrate 164 and a lateral edge. As such, the folded portion of the second substrate 164 may be connected with the garment facing surface 162c of the first substrate 162. As such, in some configurations, a fold line of the first substrate 162 and/or a fold line of the second substrate 164 may define at least a portion of the waist opening 110. It is to be appreciated that various waist configurations may be utilized. For example, as shown in Figure 3A1, the folded portion 162g may be sandwiched between the second substrate 164 and the backsheet 136. In another example shown in Figure 3A2, the second substrate 164 may be sandwiched between the folded portion 162g and the backsheet 136. Although Figures 3A1 and 3A2 show configurations of the first belt 106, it is to be appreciated that such configurations may be applied with the second belt 108.

It is to be appreciated that the first elastic belt 106 and the second elastic belt 108 may comprise the same materials and/or may have the same structure. In some embodiments, the first elastic belt 106 and the second elastic belt may comprise different materials and/or may have different structures. It should also be appreciated that components of the first elastic belt 106 and the second elastic belt 108, such as the first substrate 162, and/or second substrate 164 may be constructed from various materials. For example, the first and/or second belts may include a first substrate 162, and/or second substrate 164 that may be manufactured from materials such as plastic films; apertured plastic films; woven or nonwoven webs of natural materials (e.g., wood or cotton fibers), synthetic fibers (e.g., polyolefins, polyamides, polyester, polyethylene, or polypropylene fibers) or a combination of natural and/or synthetic fibers; or coated woven or nonwoven webs. In some configurations, the first and/or second belts may include a first substrate 162, and/or second substrate 164 comprising a nonwoven web of synthetic fibers, and may include a stretchable nonwoven. In some configurations, the first and second elastic belts may include an inner hydrophobic, non-stretchable nonwoven material and an outer hydrophobic, non-stretchable nonwoven material. It is to be appreciated that the belts may configured in various ways, such as disclosed for example, in U.S. Patent Publication No. US2022/0142828A1 and Chinese Patent Application No. CN2021/077843.

Elastic material 167 may be positioned between the wearer facing surface 162d of the first substrate 162 and the garment facing surface 164c of the second substrate 164. It is to be appreciated that the elastic material 167 may include one or more elastic elements such as strands, ribbons, elastic films, or panels extending along the lengths of the elastic belts. As shown in Figures 2A and 3, the elastic material 167 may include a plurality of elastic strands 168. In some configurations, the elastic material 167 may be an elastic film used to form a zero-strain elastic laminate comprising an elastic film bonded to one or more nonwoven layers and subsequently subjected to mechanical deformation or activation sufficient to weaken the nonwoven layer(s) and enable the laminate to stretch and recover elastically.

It is also to be appreciated that the first substrate 162, second substrate 164, and/or elastic material 167 of the first elastic belt 106 and/or second elastic belt 108 may be bonded together and/or with other components, such as the chassis 102, with adhesive and/or mechanical bonds. It is to be appreciated that adhesive and mechanical bonding methods may be utilized alone or in combination with each other.

In some configurations, adhesive may be applied to at least one of the first substrate 162, second substrate 164, and/or elastic material 167 when being combined to form the first elastic belt 106 and/or second elastic belt 108. In some configurations, mechanical bonding devices may apply mechanical bonds to the to at least one of the first substrate 162, second substrate 164, and/or elastic material 167 when being combined to form the first elastic belt 106 and/or second elastic belt 108. Such mechanical bonds may be applied with heat, pressure, and/or ultrasonic devices. In some configurations, mechanical bonding devices may apply bonds that bond the first substrate 162, second substrate 164, and/or elastic material 167 together and/or may act to trap or immobilize discrete lengths of the contracted elastic strands in the first elastic belt 106 and/or second elastic belt 108.

It is also to be appreciated that the first substrate 162, second substrate 164, and/or elastic material 167 may be bonded together with various methods and apparatuses to create various elastomeric laminates, such as described in U.S. Patent Publication Nos. 2018/0168878 A1; 2018/0168877 A1; 2018/0168880 A1; 2018/0170027 A1; 2018/0169964 A1; 2018/0168879 A1; 2018/0170026 A1; 2018/0168889 A1; 2018/0168874 A1; 2018/0168875 A1; 2018/0168890 A1; 2018/0168887 A1; 2018/0168892 A1; 2018/0168876 A1; 2018/0168891 A1; 2019/0070042 A1; 2019/0070041 A1; 2021/0282797A1; and 2021/0275362 A1, and combinations thereof.

It is to be appreciated that components of the first elastic belt 106 and/or the second elastic belt 108 may be assembled in various ways and various combinations to create various desirable features that may differ along the lateral width and/or longitudinal length of the first elastic belt 106 and/or the second elastic belt 108. Such features may include, for example, Dtex values, bond patterns, aperture arrangements, elastic positioning, Average Dtex values, Average Pre-Strain values, rugosity frequencies, rugosity wavelengths, height values, and/or contact area. It is to be appreciated that differing features may be imparted to various components, such as for example, the first substrate 162, second substrate 164, and elastic material 167 before and/or during stages of assembly of the first elastic belt 106 and/or the second elastic belt 108.

It is to be appreciated that the first elastic belt 106 and/or the second elastic belt 108 may include various configurations of belt elastic materials 167 arranged in relation to each other and to the first substrate 162, and the second substrate 164. As discussed above, the elastic material 167 may include configurations of one or more elastic elements such as strands, ribbons, films, or panels positioned in various arrangements. In some configurations, the elastic material 167 may comprise various elastics, elastic features and arrangements, and processes for assembly, such as described in 2018/0168889 A1; 2018/0168874 A1; 2018/0168875 A1; 2018/0168890 A1; 2018/0168887 A1; 2018/0168892 A1; 2018/0168876 A1; 2018/0168891 A1; 2019/0298586 A1; 2019/0070042 A1; 2018/0168878 A1; 2018/0168877 A1; 2018/0168880 A1; 2018/0170027 A1; 2018/0169964 A1; 2018/0168879 A1; 2018/0170026 A1; 2019/0070041 A1; 2021/0282797A1; and 2021/0275362 A1. It is also to be appreciated the elastic materials 167 herein may be configured with identical or different colors in various different locations on the first elastic belt 106 and/or the second elastic belt 108.

In some configurations, the elastic material 167 may be configured as elastic strands 168 disposed at a constant interval in the longitudinal direction. In other embodiments, the elastic strands 168 may be disposed at different intervals in the longitudinal direction. In some configurations, the Dtex values of the elastic strands 168 may be constant or varied along the longitudinal direction. In some configurations, the elastic material 167 in a stretched condition may be interposed and joined between uncontracted substrate layers. When the elastic material 167 is relaxed, the elastic material 167 returns to an unstretched condition and contracts the substrate layers. The elastic material 167 may provide a desired variation of contraction force in the area of the ring-like elastic belt. It is to be appreciated that the chassis 102 and elastic belts 106, 108 may be configured in different ways other than as depicted in attached Figures. It is also to be appreciated that the elastic material 167 material may be joined to the substrates continuously or intermittently along the interface between the elastic material 167 material and the substrates. In some configurations, the elastic strands 168 may be in the form of extruded elastic strands, which may also be bonded with the first substrate 162 and/or second substrate 164 in a pre-corrugated configuration, such as disclosed for example in U.S. Patent No. 5,681,302,

As discussed above for example with reference to Figures 2A and 3, the elastic material 167 discussed herein may be in the form of elastic strands 168. In some configurations, the elastic strands 168 may be parallel with each other and/or with the lateral axis 126. It is to be appreciated that the first elastic belt 106 and/or second elastic belt 108 may be configured to include various quantities of elastic strands 168. In some configurations, elastic strands 168 may be grouped in pairs. In some configurations, the first elastic belt 106 and/or second elastic belt 108 may comprise from about 10 to about 1500 elastic strands 168. It is also to be appreciated that elastic strands 168 herein may comprise various Dtex values, strand spacing values, and pre-strain values and such elastic strands 168 may utilized with other elastic strands to create first and second elastic belts 106, 108 comprising elastic strands 168 in various combinations of Dtex values, strand spacing values, and pre-strain values. For example, in some configurations, the Average-Dtex of one or more elastic strands 168 may be greater than 500. In some configurations, the Average-Dtex of one or more elastic strands 168 may be from about 10 to about 1500, specifically reciting all 1 Dtex increments within the above-recited range and all ranges formed therein or thereby. In some configurations, a plurality of elastic strands 168 may comprise an Average-Strand-Spacing of less than or equal to 4 mm. In some configurations, a plurality of elastic strands 168 may comprise an Average-Strand-Spacing from about 0.25 mm to about 4 mm, specifically reciting all 0.01 mm increments within the above-recited range and all ranges formed therein or thereby. In some configurations, a plurality of elastic strands 168 may comprise an Average-Strand-Spacing of greater than 4 mm. In some configurations, the Average-Pre-Strain of each of a plurality of elastic strands may be from about 50% to about 400%, specifically reciting all 1% increments within the above-recited range and all ranges formed therein or thereby. In some configurations, the elastic strands 168 comprise an Average-Strand-Spacing from about 0.25 mm to about 4 mm and an Average-Dtex from about 10 to about 500. In some configurations, the elastic strands 168 may comprise an Average-Pre-Strain from about 75% to about 300%.

In some configurations, a first plurality of elastic strands may comprise a first Average-Pre-Strain from about 75% to about 300%, and a second plurality of elastic strands may comprise a second Average-Pre-Strain that is greater than first Average-Pre-Strain. In some configurations, a first plurality of elastic strands comprises an Average-Strand-Spacing from about 0.25 mm to about 4 mm and an Average-Dtex from about 10 to about 500; and a second plurality of elastic strands may comprise an Average-Strand-Spacing greater than about 4 mm and an Average-Dtex greater than about 450.

In some configurations, such as shown in Figure 2A, the elastic strands 168 may be referred to herein as outer waist elastics 170 and inner waist elastics 172. Elastic strands 168, such as the outer waist elastics 170, may continuously extend laterally between the first and second opposing end regions 106a, 106b of the first elastic belt 106 and between the first and second opposing end regions 108a, 108b of the second elastic belt 108. Some elastic strands 168, such as the inner waist elastics 172, may be configured with discontinuities in areas, such as for example, where the first and second elastic belts 106, 108 overlap portions of the chassis 102, such as the absorbent assembly 140.

As shown in Figure 2A, the first elastic belt 106 and/or the second elastic belt 108 may be configured with low-stretch zones 701 and high-stretch zones 703. The first elastic belt 106 and/or the second elastic belt 108 may include a first high-stretch zone 703a and a second high-stretch zone 703b separated laterally by a low-stretch zone 701. Portions of the chassis 102, such as the backsheet 136 and absorbent assembly 140, may be connected with the first elastic belt 106 and/or the second elastic belt 108 in the low-stretch zones 701 in the first waist region 116 and/or the second waist region 118. The high-stretch zones 703 are elasticated by the elastic material 167, such as the elastic strands 168, 172; and the low-stretch zones 701 may comprise cut lines separating the elastic material 167, such as the elastic strands 168, 172. In some configurations, the elastic material 167 may be cut in an unbonded region where the elastic material is not bonded with first substrate 162 and the second substrate 164. Thus, the elastic material 167 retracts from the unbonded region and form low-stretch zone 701. In some configurations, the elastic material 167 may be cut into several discrete pieces. In turn, the low-stretch zones 701 define regions of the first elastic belt 106 and/or the second elastic belt 108 that have relatively less elasticity than the high-stretch zones 703. The discrete elastic material 167 that has been cut and which are elastically contracted do not add any substantial amount of elastication to the low-stretch zone 701. As such, upon application of a force, the high-stretch zones 703 will elongate more than the low-stretch zones 701. As provided above, the terms "elastic," "elastomer" or "elastomeric" refers to materials exhibiting elastic properties, which include any material that upon application of a force to its relaxed, initial length can stretch or elongate to an elongated length more than 10% greater than its initial length and will substantially recover back to about its initial length upon release of the applied force. In some configurations, the first elastic belt 106 and/or the second elastic belt 108 may be configured with high-stretch zones 703 that are elastic and may be configured with low-stretch zones 701 that are not elastic or "inelastic."

As discussed above, the diaper pants 100P described with reference to Figures 1-3C may include one or more frangible pathways in the first belt 106 and/or the second belt 108. For example, Figures 4A-4C show an example diaper pant 100P with a first belt 106 that includes frangible pathways 700. The frangible pathways 700 may be configured to allow the first elastic belt 106 to be relatively easily torn along the frangible pathway 700, such as when removing the diaper pant 100P from a wearer. Figure 4B shows a view of the diaper pant 100P from Figure 4A, illustrating the first belt 106 after having been torn along the frangible pathway 700 through both the outer longitudinal outer laterally extending edge 107a and the inner laterally extending edge 107b of the first belt 106. As such, the first elastic belt 106 shown in Figure 4B is separated by opposing tear lines 705. It is to be appreciated the first elastic belt 106 may be torn along both frangible pathways 700 in Figures 4B. For example, Figure 4C shows the diaper pant of Figure 4A showing the front belt having been torn along two frangible pathways 700. As shown in Figure 4C, the central region 106c of the first elastic belt 106 may remain bonded with the chassis 102 after separating the first and second opposing end regions 106a, 106b from the central region 106c by tearing the elastic belt 106 along the frangible pathways 700.

As discussed in more detail below, the frangible pathways 700 comprise a plurality of lines of weakness 704 configured such that all elastic strands 168 in the first elastic belt 106 are severed at least once in the frangible pathway 700. Severing the elastic strands 168 in the frangible pathway 700 helps make it relatively easier to tear the first elastic belt 106 along the frangible pathway 700. For example, when the elastic strands 168 are severed, the first substrate 162 and second substrate 164 of the first elastic belt 106 need only need to be torn without having to also tear uncut elastic strands 168. It is to be appreciated that the diaper pant 100P may include various quantities of frangible pathways 700 that may be: positioned in various locations; define various shapes; and extend for various lengths. For example, the first elastic belt 106 may comprise a first belt length defined by a longitudinal distance between the proximal edge 107b and the distal edge 107a, and the frangible pathway 700 may extend for a total length from an outermost edge of a line of weakness 704 nearest the proximal edge 107b of the first belt 106 to an outermost edge of a line of weakness 704 nearest the distal edge 107a of the first belt 106. In some configurations, the frangible pathway 700 may extend for a total length that is greater than, equal to, or less than the first belt length. In some configurations, the lines of weakness 704 may extend for a length from a first end to a second end, and a sum of the all the lengths of lines of weakness 704 in the frangible pathway 700 may be greater than the frangible pathway total length.

In some configurations, diaper pants 100P may be configured such that one or both of the first elastic belt 106 and the second elastic belt 108 include one or more frangible pathways 700. The frangible pathways 700 may be positioned in various locations on the first and second elastic belts 106, 108. For example, such as shown in Figures 4A-4C, frangible pathways 700 may extend to overlap with the chassis 102. In some configurations, the frangible pathways 700 may extend in straight lines and/or may be curved and/or have curved portions. In some configurations, the frangible pathways 700 may extend longitudinally for the entire length or less than the entire length of the first belt 106 and/or second belt 108. In some configurations, frangible pathways 700 may be positioned partially or entirely laterally between the first and second side seams 178, 180 and the chassis 102.

In some configurations, the frangible pathways 700 may be configured and/or positioned to provide access to and/or function with other features, such as disposal features. For example, the diaper pant 100P shown in Figures 4A-4C includes fastener components 707 positioned on the wearing facing surface 115b of the first elastic belt 106. In some configurations, the fastener components 707 may be positioned between the first elastic belt 106 and the chassis 102. The fastener component 707 may be configured to refastenably connect with other portions of the diaper pant 100P, such as for example, the garment facing surfaces of the first elastic belt 106, the second elastic belt 108, or the chassis 102. As such, once the first elastic belt 106 is torn along the frangible pathways 700, the diaper pant 100P may be removed from a wearer and rolled or folded up for disposal, and the fastener component 707 may be connected with another portion of the diaper parit 100P to help maintain the diaper pant 100P in a disposal configuration. For example, Figure 4C shows a diaper pant 100P after tearing the first elastic belt 106 along two frangible pathways. Figure 5A shows the diaper pant 100P of Figure 4C with the chassis 102 being rolled up onto itself in a longitudinal direction. And Figure 5B shows the diaper pant 100P of Figure 5A with fastener components 707 refastenably connected with the backsheet 136 of the chassis 102 to maintain the diaper pant 100P in a disposal configuration. In some configurations, when tearing the elastic belt along the frangible pathway 700, the tearing process may begin by tearing from the outer edge 107a. or the inner edge 107b of the elastic belt 106. As discussed in more detail below, in some configurations, the first elastic belt 106 may also include an opening, such as a slit located adjacent to or in the proximity of the fastener component 707 and the weakened region 700 to help facilitate starting to tear the frangible pathway 700 in a region of the elastic belt 106 longitudinally between the outer edge 107a and the inner edge 107b.

It is also to be appreciated that the fastener component 707 may be configured in various ways, such as hooks, loops, and/or adhesive. For example, the fastener component 707 may comprise hook elements or adhesive adapted to refastenably connect with another surface of the diaper pant 100P. In some configurations, the fastener component 707 may comprise loop elements adapted to refastenably connect with hook surface on the diaper pant 100P. The fastener component 707 may be a separate element connected with the elastic belt 106 in various ways, such as mechanical bonding, adhesive bonding, or both. In some configurations, the fastener component 707 may be integrally formed from materials of the elastic belt 106, 108.

As previously mentioned, the fastener component 707 may comprise a hook material that can refastenably engage with substrates, such as nonwovens for example, on an exterior surface of the diaper pant 100P. For example, the fastener component 707 may comprise a substrate comprising hooks, with the substrate bonded to the elastic belt 106, 108, such as the second substrate 164, which may be in the form of a nonwoven. It is to be appreciated that the substrate may be bonded to the elastic belt 106, 108 in various ways, such as for example, with mechanical bonds, thermal bonds, ultrasonic bonds, and/or adhesive bonds or combinations thereof. In some configurations, hooks may be integrally formed from the second substrate 164, which may be in the form of a nonwoven. The fastener component 707 may comprise one material or a combination of two or more materials arranged in at least partially overlapping configuration. In some configurations, the fastener component 707 may comprise other fastener types as known in the art.

It is to be appreciated that the fastener component 707 may comprise any of a wide variety of shapes, including rectangles or other polygons, circles, ovals, shapes having exterior convexities or concavities or combinations thereof, or one or a plurality of lines or geometric shapes forming an array. It is to be appreciated that the fastener component 707 may comprise various sizes. For example, in some configurations, the fastener component 707 may have a lateral width of between about 5 mm and about 100 mm, specifically reciting all 0.1 mm increments within the above-recited range and all ranges formed therein or thereby. In some configurations, the fastener component 707 may have a longitudinal length of between about 10 mm and about 100 mm, specifically reciting all 0.1 mm increments within the above-recited range and all ranges formed therein or thereby. The fastener component 707 may be aligned parallel the lateral centerline 126a, 126b of the elastic belt 106, 108 or may be oriented at an angle relative the longitudinal centerline 126a, 126 of the elastic belt 106, 108 of between 0 and 90 degrees. The fastener component 707 may comprise an array of two or more spaced-apart fastening elements.

It is also to be appreciated that the frangible pathways 700 may comprise lines of weakness 704 that are: configured in various ways, positioned in various locations and orientations relative to each other; defined by various shapes; and extend for various lengths. For example, in some configurations, the lines of weakness 704 comprise discrete cut lines that penetrate through some or all the layers of the elastic belt 106. In some configurations, the lines of weakness 704 comprise discrete bonds wherein materials of the first substrate and the second substrate are fused together. In some configurations, the lines of weakness 704 may be linear, curvilinear, or have a regular or irregular geometry and may comprise one or more of a perforation, a bond, an aperture, or a mechanically thinned region of a material such as a nonwoven, or a combination thereof. It is also to be appreciated that the lines of weakness 704 can be formed with different lengths and spacings to achieve different separation forces.

As discussed above, absorbent articles 100, such as diaper pants 100P, may be configured with frangible pathways 700 comprising lines of weakness 704 arranged in various ways to help improve a caregiver's ability to remove a soiled diaper pant 100P from a wearer without having to remove a soiled diaper pant from a wearer by sliding the soiled diaper pant down the wearer's legs. As discussed above, the frangible pathways 700 may be configured to allow the first elastic belt 106 and/or the second elastic belt 108 to be relatively easily torn along the frangible pathway 700, such as when removing the diaper pant 100P from a wearer. In addition, the frangible pathways 700 may also be configured to provide access to fastener components 707 that may be used to help hold a soiled product in a disposal configuration. The following provides a discussion of example implementations of frangible pathways 700 on diaper pants 100P in the context of the above description of various details of absorbent articles 100, fastener components 707, frangible pathways 700, and lines weakness 704. It is to be appreciated that discussions of frangible pathways 700 in the first elastic belt 106 herein may also apply to frangible pathways 700 in the second elastic belt 108.

It is to be appreciated that frangible pathways 700 may be positioned in various locations and/or orientations relative to other components of the absorbent article 100 and/or may be configured to function in various ways to help facilitate removal of diaper pant from a wearer. For example, the diaper pant 100P shown in Figures 6A and 6B may include one or more frangible pathways 700 extending between a distal terminus 808 on the outer edge 107a of the first belt 106 and a distal terminus 810 on the inner edge 107b of the first belt 106. As illustrated in Figures 6A and 6B, the diaper pant 100P includes a first frangible pathway 700a and a second frangible pathway 700b in the first belt 106. The first frangible pathway 700a may extend between a first distal terminus 808a on the outer edge 107a of the first belt 106 and a first proximal terminus 810a on the inner edge 107b of the first belt 106. And the second frangible pathway 700b may extend between a second distal terminus 808b on the outer edge 107a of the first belt 106 and a second proximal terminus 810b on the inner edge 107b of the first belt 106. It is to be appreciated that the first and second frangible pathways 700a, 700b may comprise lines of weakness 704 as described above.

It is to be appreciated that the first distal terminus 808a and the second distal terminus 808b may be located in various lateral positions on the outer edge 107a of the first belt 106. For example, in some configurations, the first distal terminus 808a and/or the second distal terminus 808b may be positioned in the central region 106c of the first belt 106. In some configurations, the first distal terminus 808a and/or the second distal terminus 808b may be positioned laterally between the first longitudinal edge 128 and the second longitudinal edge 130 of the chassis 102. In some configurations, the first distal terminus 808a and/or the second distal terminus 808b may be positioned in the first end region 106a and/or the second end region 106b of the first belt 106. In some configurations, the first distal terminus 808a and/or the second distal terminus 808b may be positioned laterally outboard of the first longitudinal edge 128 and the second longitudinal edge 130 of the chassis 102. In some configurations, the first distal terminus 808a and/or the second distal terminus 808b may be positioned laterally between the first longitudinal edge 128 of the chassis 102 and the first side seam 178 and/or may be positioned laterally between the second longitudinal edge 130 of the chassis 102 and the second side seam 180. In some configurations, the first distal terminus 808a may be laterally aligned with the first longitudinal edge 128 of the chassis 102 or the first longitudinal side edge 111a of the first belt 106. In some configurations, the first distal terminus 808a may be positioned laterally between the first longitudinal edge 128 of the chassis 102 and the first longitudinal side edge 111a of the first belt 106. In some configurations, the second distal terminus 808b may be laterally aligned with the second longitudinal edge 130 of the chassis 102 or the second longitudinal side edge 111b of the first belt 106. In some configurations, the second distal terminus 808b may be positioned laterally between the second longitudinal edge 130 of the chassis 102 and the second longitudinal side edge 111b of the first belt 106.

It is also to be appreciated that the first proximal terminus 810a and the second proximal terminus 810b may be located in various lateral positions on the inner edge 107b of the first belt 106. For example, in some configurations, the first proximal terminus 810a and/or the second proximal terminus 810b may be positioned in the central region 106c of the first belt 106. In some configurations, the first proximal terminus 810a and/or the second distal terminus 810b may be positioned laterally between the first longitudinal edge 128 and the second longitudinal edge 130 of the chassis 102. In some configurations, the first proximal terminus 810a and/or the second proximal terminus 810b may be positioned in the first end region 106a and/or the second end region 106b of the first belt 106. In some configurations, the first proximal terminus 810a and/or the second proximal terminus 810b may be positioned laterally outboard of the first longitudinal edge 128 and the second longitudinal edge 130 of the chassis 102. In some configurations, the first proximal terminus 810a and/or the second proximal terminus 810b may be positioned laterally between the first longitudinal edge 128 of the chassis 102 and the first side seam 178 and/or may be positioned laterally between the second longitudinal edge 130 of the chassis 102 and the second side seam 180. In some configurations, the first proximal terminus 810a may be laterally aligned with the first longitudinal edge 128 of the chassis 102 or the first longitudinal side edge 111a of the first belt 106. In some configurations, the first proximal terminus 810a may be positioned laterally between the first longitudinal edge 128 of the chassis 102 and the first longitudinal side edge 111a of the first belt 106. In some configurations, the second proximal terminus 810b may be laterally aligned with the second longitudinal edge 130 of the chassis 102 or the second longitudinal side edge 111b of the first belt 106. In some configurations, the second proximal terminus 810b may be positioned laterally between the second longitudinal edge 130 of the chassis 102 and the second longitudinal side edge 111b of the first belt 106.

It is to be appreciated that the first distal terminus 808a and the second distal terminus 808b may be located in various longitudinal positions between the outer edge 107a and the inner edge 107b of the first belt 106. And the first proximal terminus 810a and the second proximal terminus 810b may be located in various longitudinal positions between the outer edge 107a and the inner edge 107b of the first belt 106. For example, in some configurations, such as shown in Figure 6B1 for example, the first distal terminus 808a and/or the first proximal terminus 810a may be located on the first side seam 178 at positions longitudinally inboard of the outer edge 107a and longitudinally outboard of the inner edge 107b of the first belt 106. Also, as shown in Figure 6B1, the second distal terminus 808b and/or the second proximal terminus 810b may be located on the second side seam 180 at positions longitudinally inboard of the outer edge 107a and longitudinally outboard of the inner edge 107b of the first belt 106. As such, completing the tearing process of the first belt 106 may also require tearing portions of the first and/or second side seams 178, 180.

With continued reference to Figure 6B, the first belt 106 may also comprise grip regions 801 providing a place where a user may grasp a portion of the first belt 106 and begin the process of tearing the first belt along the frangible pathway 700. The grip region 801 may comprise an accessibility opening 802 in the first belt 106 and may also comprise a fastener component 707 positioned adjacent the accessibility opening 802. The accessibility opening 802 may comprise slits and/or openings in the first belt 106 and may penetrate through some or all layers of the first belt 106. It is to be appreciated that such slits or openings may be curved and/or straight. The accessibility opening 802 may also be considered part of the frangible pathway 700.

As shown in Figure 6B, the diaper pant 100P may include a first grip region 801a including a first accessibility opening 802a and second grip region 801b including a second accessibility opening 802b in the first belt 106. The first and second accessibility openings 802a, 802b may be positioned between the outer edge 107a and the inner edge 107b of the first belt 106. The first and second accessibility openings 802a, 802b may also be positioned in the central region 106c of the first belt 106 and may be positioned between the first longitudinal edge 128, the second longitudinal edge 130 of the chassis 102, and the first lateral edge 144 of the chassis 102. In addition, a first fastener component 707a may be positioned adjacent the first accessibility opening 802a, and a second fastener component 707a may be positioned adjacent the second accessibility opening 802a. The first frangible pathway 700a comprises a first tear zone 813a extending from the first accessibility opening 802a to the first distal terminus 808a and a second tear zone 813b extending from the first accessibility opening 802a to the first proximal terminus 810a. The second frangible pathway 700b comprises a first tear zone 813a extending from the second accessibility opening 802b to the second distal terminus 808b and a second tear zone 813b extending from the second accessibility opening 802b to the second proximal terminus 810b. As discussed in more detail below, the accessibility opening 802 may help provide a caregiver or wearer access to and/or to grasp the fastener component 707 in the grip region 801 with a finger or thumb. The caregiver or user may then pull on grip region 801 to begin tearing the first belt 106 on the frangible pathway 700. In some configurations, tear lines may simultaneously propagate along the first tear zone 813a and the second tear zone 813b laterally outward from the central region 106c of the first belt 106 toward the distal terminus 808 and the proximal terminus 810. It is also to be appreciated that some diaper pants 100P herein may be configured to include a frangible pathway 700 that extends through or around the fastener component 707 without an accessibility opening. In turn, a user may pinch and/or pull the belt where the frangible pathway 700 is located at or adjacent the fastener component 707 to initiate the tearing process along the frangible pathway 700.

As shown in Figure 6B, the frangible pathways 700 may be configured to extend laterally inward from the from the distal terminus 808 and/or the proximal terminus 810. In turn, portions of the frangible pathway 700 may extend to define an angle that is less than 90 degrees with respect to the outer edge 107a and/or the inner edge 107b of the first belt 106. Thus, the frangible pathway may define an overall length that is greater than a longitudinal length LT1 of the first belt 106 and/or the longitudinal length LT2 of the second belt 108 discussed above with reference to Figures 2C-2E.

It is to be appreciated that grip regions 801 and accessibility openings 802 may be located in various positions in the first end region 106a, the second end region 106b, and/or the central region 106c of the first belt 106. Grip regions 801 and accessibility openings 802 may be positioned between the first longitudinal side edge 111a, the second longitudinal side edge 111b, the outer edge 107a, and the inner edge 107b of the first belt 106. For example, the first accessibility opening 802a and/or the second accessibility 802b may be entirely laterally positioned between the first longitudinal edge 128 and the second longitudinal edge 130 of the chassis 102. In some configurations, the first accessibility opening 802a may be positioned laterally between the first longitudinal side edge 128 of the chassis 102 and the first longitudinal side edge 111a of the first belt 106 and/or first side seam 178. In some configurations, the second accessibility opening 802b may be positioned laterally between the second longitudinal side edge 130 of the chassis 102 and the second longitudinal side edge 111b of the first belt 106 and/or second side seam 180. In some configurations, the first accessibility opening 802a and/or the second accessibility opening 802b may be positioned longitudinally between the first lateral edge 144 of the chassis 102 and the inner edge 107b of the first belt 106 and/or may be positioned longitudinally between the first lateral edge 144 of the chassis 102 and the outer edge 107a of the first belt 106. In some configurations, the first accessibility opening 802a may extend across the first longitudinal edge 128 and/or the first lateral edge 144 of the chassis 102, and/or the second accessibility opening 802b may extend across the second longitudinal edge 130 and/or the first lateral edge 144 of the chassis 102.

It is also be appreciated that accessibility openings 802 may be located in various positions relative to fastener components 707. For example, in some configurations, the accessibility opening 802 may be positioned longitudinally between the fastener component 707 and the outer edge 107a of the first belt 106. In some configurations, the accessibility opening 802 may be positioned longitudinally between the fastener component 707 and the inner edge 107b of the first belt 106. In some configurations, the accessibility opening 802 may be positioned laterally inboard of the fastener component 707. It is also to be appreciated that more than one accessibility opening 802 may be located adjacent a fastener component 707. As discussed in more detail below, the accessibility opening 802 also be configured to extend partially or entirely through a fastener component 707 and may divide a fastener component 707 into two or more parts.

As mentioned above, the accessibility opening 802 may comprise slits and/or openings in the first belt 106 and may be curved and/or straight. It is to be appreciated that the accessibility openings 802 may also be oriented in various ways. For example, the accessibility opening 802 may be generally oriented perpendicularly relative to the outer edge 107a and/or the inner edge 107b of the first belt 106. In some configurations, the accessibility opening 802 may be generally oriented parallel relative to the outer edge 107a and/or the inner edge 107b of the first belt 106. In some configurations, the accessibility opening 802 may comprise a slit that extends along a line in a lateral direction to define an angle from about 0 degrees to about 45 degrees with respect to the outer edge 107a and/or the inner edge 107b of the first belt 106, specifically reciting all 1 degree increments within the above-recited range and all ranges formed therein or thereby. In some configurations, the accessibility opening 802 may define a length dimension in the range of about 5 mm to about 50 mm, specifically reciting all 0.1 mm increments within the above-recited range and all ranges formed therein or thereby.

As discussed above, the diaper pant 100P may include one or more fastener components 707 adapted to refastenably connect with at least one other component of the diaper pant 100P in a disposal configuration. It is to be appreciated that the fastener components 707 may be configured in various shapes and sizes, and may be located in various positions relative to other components of the diaper pant 100P. As shown in Figure 7A, the fastener components 707 may comprise a lateral centerline 126d oriented substantially parallel to the lateral centerline 126a of the first elastic belt 106 and/or the lateral centerline 126b of the second elastic belt 108 and/or the lateral centerline 126c of the chassis 102. The fastener components 707 may comprise a longitudinal centerline 124d oriented substantially parallel to the longitudinal centerline 124a of the first elastic belt 106 and/or the longitudinal centerline 124b of the second elastic belt 108 and/or the longitudinal centerline 124c of the chassis 102. With continued reference to Figure 7A, the fastener component 707 may also comprise an inboard lateral edge 708, an outboard lateral edge 709, an inboard longitudinal edge 710, and an outboard longitudinal edge 711.

As shown in Figure 7AA1, in some configurations, fastener components 707 may be positioned on and connected with the wearer facing surface 115b of the first elastic belt 106 and/or the second elastic belt 108 in a region where the first elastic belt 106 and/or second elastic belt 108 overlaps the chassis 102. In some configurations, the fastener component 707 may be sandwiched between the second substrate 164 of the first elastic belt 106 or the second elastic belt 108 and the backsheet 136 of the chassis 102. In some configurations, such as shown in Figure 7AA1, the fastener component 707 comprises hooks 715 protruding from a base 717, and the hooks 715 extend from the first belt 106 toward the backsheet 136. The fastener component 707 may be configured as a separate discrete element that may be connected with the wearer facing surface 115b of the first belt 106 in various ways. For example, as shown in Figure 7AA1, adhesive 716 may connect the base 717 of the fastener component 707 with wearer facing surface 115b of the first belt 106. It is to be appreciated that the fastener component 707 may be connected with the first belt 106 by mechanical bonding in addition to or instead of adhesive. It is to be appreciated that the base 717 may be configured in various ways. For example, the base 717 may comprise a thermoplastic film. In some configurations, the base 717 may comprise a laminate with various layers bonded together, such as disclosed for example in U.S. Patent Publication No. 2021/0045931A1. For example, the base 717 may comprise a thermoplastic film layer bonded with a nonwoven layer. It is to be appreciated that such layers may be bonded together in various ways, such as with adhesive, mechanical bonding, and/or extrusion bonding. In some configurations, the fastener component 707 may be integrally formed from materials of the first belt 106, such as shown for example in Figure 7AA2, or may be integrally formed from materials and attached with the first belt.

As shown for example in Figure 6B, a portion of the chassis 102 may overlap the inner wearer facing surface 115b of the first belt 106 to define a chassis overlap region 850. As such, the chassis overlap region 850 may extend laterally between the first longitudinal edge 128 and the second longitudinal edge 130 of the chassis 102 and longitudinally between the first lateral edge 144 of the chassis 102 and the inner edge 107b of the first belt 106. To help prevent contact of the fastener component 707 with a wearer's skin while wearing the diaper pant 100P, the fastener components 707 may be positioned on and connected with the wearer facing surface 115b of the first elastic belt 106 and/or the wearer facing surface 117b of the second elastic belt 108 in the chassis overlap region 850 where the first elastic belt 106 and/or second elastic belt 108 overlaps the chassis 102. For example, the fastener component 707 may be sandwiched between the wearer facing surface 115b of the first belt 106 and the chassis 102. In some configurations, the fastener component 707 may be sandwiched between the second substrate 164 of the first elastic belt 106 or the second elastic belt 108 and the backsheet 136 of the chassis 102. In some configurations, the fastener component 707 may be positioned laterally between the first longitudinal side edge 128 and the second longitudinal side edge 130 of the chassis 102. The fastener component 707 may also be positioned longitudinally between the first lateral edge 144 of the chassis 102 and the inner edge 107b of the first belt 106. As shown in Figure 7A, the fastener component 707 may be positioned adjacent the frangible pathway 700. The accessibility opening 802, which may be considered part of the frangible pathway 700, may be positioned adjacent the fastener component 707. As such, the frangible pathway 700 may partially surround the fastener component 707. In some configurations, such as shown in Figure 7B, the frangible pathway 700 may extend through the fastener component 700, effectively dividing the fastener component 707 into a first fastener part 707' and a second fastener part 701".

As discussed above, the backsheet 136 of the chassis 102 may comprise a laminate comprising a film layer and a nonwoven layer. In some configurations, the nonwoven layer may completely cover the film layer to define the garment facing surface 134 of the chassis 102. In some configurations, the nonwoven layer may partially cover the film layer, and as such, both the film layer and the nonwoven layer may define the garment facing surface 134 of the chassis 102. For example, in some configurations, the film layer may extend entirely between the inner edge 107b of the first belt 106 and the first lateral edge 144 of the chassis 102, and the nonwoven layer may also extend entirely between the inner edge 107b of the first belt 106 and the first lateral edge 144 of the chassis 102 to completely cover the film layer and define the garment facing surface 134 of the chassis 102 in the overlap region 850.

In some configurations, a portion of the nonwoven layer of the backsheet 136 may be positioned between the fastener components 707 and the first belt 106. For example, as discussed above with reference to Figures 7AA1 and 7AA2, the fastener component 707 may comprise hooks 715 that extend toward the backsheet 136 of the chassis 102. As such, the hooks of the fastener component 707 may be releasably or refastenably connected with the nonwoven layer of the backsheet 136. In some configurations, the hooks of the fastener component 707 may not be releasably or refastenably connected with the film layer of the backsheet 136. In some configurations, the fastener component 707 may comprise a pressure sensitive adhesive instead of hooks, and as such, the pressure sensitive adhesive may releasably connect the fastener component 707 with the backsheet. In some configurations, such a pressure sensitive adhesive may be configured to releasably or refastenably connect the first belt 106 with a nonwoven layer and/or a film layer of the backsheet 136.

As discussed above, the position and/or location of the fastening component 707 with respect to the first belt 106 and/or second belt 108, the accessibility opening 802, and/or frangible pathway 700 may be configured to help conceal the fastener component 707 from the wearer while wearing the diaper pant 100P while also helping a caregiver successfully determine the location of and access the concealed disposal fastener component 707. For example, as discussed above, the fastener component 707 may be sandwiched between the first belt 106 and the chassis 102, and as such, is concealed or hidden while the diaper pant 100P is worn.

In some configurations, the accessibility opening 802 may be positioned so as to be more likely to be out of a wearer's range of vision and less physically accessible to the wearer's hands. In some configurations, at least a portion of the accessibility opening may be located at, or near, the inboard lateral edge 708, the outboard lateral edge 709, and/or the inboard longitudinal edge 710 of the fastener component 707. In some configurations, at least a portion of the accessibility opening may be located within the inboard lateral edge 708, the outboard lateral edge 709, the inboard longitudinal edge 710, and the outboard longitudinal edge 711 of the fastener component 707. As shown in Figures 6B and 7A, the accessibility opening 802 may be positioned in the chassis overlap region 850 adjacent fastener component 707. In some configurations, the accessibility opening 802 may be positioned between the inboard lateral edge 708 of the fastener component 707 and the inner edge 107b of the first belt 106. In some configurations, the accessibility opening 802 may extending about 5 mm or less inboard or outboard from the inboard lateral edge 708 of the fastener component 707, specifically reciting all 0.1 mm increments within the above-recited range and all ranges formed therein or thereby. In some configurations, an accessibility opening 802 in the form of a laterally extending slit may be covered and/or hidden by folds and/or wrinkles in the first belt 106 that sometimes form when wearing the diaper pant 100P. In some configurations, the accessibility opening 802 may be disposed at an angle relative to the lateral centerline 126a to help reduce the probability of the accessibility opening 802 being entirely located within a wrinkle or fold during the wearing process. In some configurations, such an angled accessibility opening 802 may not be readily visible from the perspective of a wearer.

In some configurations, the accessibility opening 802 may comprise mechanical aids and/or may be oriented at specific angles that are arranged in specific ways that help increase ergonomic and easy accessibility for a caregiver, while presenting difficulty for a wearer to operate and/or access. For example, a caregiver may be using the accessibility opening 802 while in a facing relationship to the accessibility opening 802 on the front of a diaper pant 100P, allowing a caregiver's finger or thumb to be easily slid under the fastener component 707 for removal. In contrast, a wearer would have to operate the accessibility opening 802 in a nonfacing relationship to the front of the diaper pant 100P, and the angles that a user's hand would need to achieve to operate the accessibility opening 802 may be relatively difficult, especially for younger children, toddlers, or babies. For example, a caregiver may have a better angle than the wearer to access accessibility openings 802 that are configured to generally require accessing from the inboard lateral edge 708 of the fastener component 707, or from within the fastener component 707.

In some configurations, a diaper pant 100P may not include an accessibility opening 802. For example, a frangible pathway 700 may surround the fastener component 707. In turn, the fastener component 707 may be pinched or pulled to sever the frangible pathway 700 around the fastener component 707 without the need to have an accessibility opening 802. The actions needed to sever the frangible pathway 700 without an accessibility opening 802 may be relatively more complex to do, and therefore, may present additional difficulty for a wearer to operate, while still being accessible for the caregiver. In some configurations, a frangible pathway 700 that surrounds the fastener component 707 may be configured to operate in conjunction with a relatively small accessibility opening 802 that is contained within the fastener component 707, and thus, may be used as a lift tab to sever the surrounding frangible pathway. In some configurations, the relatively small accessibility opening 802 may extend longitudinally inward or outward and/or laterally inward or outward, and/or may be oriented in such a way that would present difficulties for a wearer to access while being relatively easy for a caregiver to access.

In another configuration shown in Figures 7C, 7D, and 7E, both the frangible pathway 700 and the accessibility opening 802 may extend through the fastener component, effectively dividing the fastener component 707 into a first fastener part 707' and a second fastener part 707". The accessibility openings 802 shown in Figure 7C may comprise slits that are generally oriented in a longitudinal direction. In addition, the accessibility opening 802 extends through the fastener component 707 and may be positioned entirely within a perimeter of the fastener component. It is to be appreciated that such slits may be straight and/or curved. In some configurations, a longitudinally extending accessibility opening 802 may define a length dimension in the range of about 10 mm to about 30 mm, specifically reciting all 0.1 mm increments within the above-recited range and all ranges formed therein or thereby. In addition, in some configurations, a longitudinally extending accessibility opening 802 may also be curved to extend laterally in the range of about 2 mm to about 20 mm, specifically reciting all 0.1 mm increments within the above-recited range and all ranges formed therein or thereby.

In another configuration shown in Figure 7F, the diaper pant 100P may comprise one fastener component 707 joined to the wearer facing surface 115b of the first belt 106 in a location overlapping the longitudinal centerline 124c of the chassis 102. The longitudinal centerline 124d of the fastener component 707 may be coincident with, or in proximity of, the longitudinal centerline 124c of the chassis 102. The frangible pathway 700 may divide fastener component 707 into the first fastener component 707a and the second fastener component 707b of substantially similar size and geometry. An accessibility opening 802 may be disposed at, or in proximity of, a longitudinally inboard lateral edge 708 of the fastener component 707. Longitudinally outboard the lateral edges of the fastener component 707, the frangible pathway 700 may extend in longitudinal and lateral directions to the waist edge 121 and inner edge 107b of the first belt 106. A caregiver or wearer may access and grasp the fastener component 707 through the accessibility opening 802 and subsequently separate the frangible pathway 700 into the first and second fastener components 707a, 707b.

As discussed above, the first elastic belt 106 and/or the second belt 108 may be relatively easily torn along the frangible pathway 700, such as when removing the diaper pant 100P from a wearer. As discussed below with reference to Figures 6A-6F, frangible pathway 700 may be configured to allow a caregiver or wearer to initiate and/or completely tear the first belt 106 and/or the second belt 108 with one hand when removing a diaper pant 100P from a wearer. In addition, the first belt 106 may be separable along the first frangible pathway 700a and the second frangible pathway 700b to define a first belt zone 831, a second belt zone 832, and a third belt zone 833 positioned laterally between the first and second belt zones 831, 832.

Referring now to Figures 6A and 6B, when removing a diaper pant 100P from a wearer, a user may grab the first belt 106 in the grip region 801 by inserting one or more fingers and/or a thumb through the accessibility opening 802 to grasp a portion of the first 106 and fastener component 707. For example, with reference to Figures 6B and 6C, a caregiver may insert a finger or thumb through the first accessibility opening 802a and grasp the first belt 106 and the first fastener component 707a with a first hand. The caregiver's opposing second hand may be used to help stabilize the wearer. For example, the caregiver's opposing second hand may apply a holding or stabilizing force to the wearer at the central region 106c of the first belt 106. The user's first hand may then exert a pulling force F on the first grip region 801a of the first belt 106 outward away from the wearer to initiate a tearing of the first belt 106 along the first frangible pathway 700a, such as shown in Figure 6C.

With continued reference to Figure 6C, a force F generally represented by an arrow is applied to the first grip region 801a in a direction generally toward the first end region 106a of the first belt 106 and/or outward away from the first belt 106 and the wearer. As the force F is applied, a first tear line 705a and a second tear line 705b may simultaneously propagate along the first tear zone 813a and the second tear zone 813b, respectively. The first tear line 705a may propagate from the first accessibility opening 802a along the first tear zone 813a of the first frangible pathway 700a in longitudinal and lateral directions around the first fastener component 707a and then in a direction D1 that is generally laterally and longitudinally outward from the central region 106c of the first belt 106 and toward the first distal terminus 808a in the first end region 106a of the first belt 106. Simultaneously, the second tear line 705b may propagate from the first accessibility opening 802a along the second tear zone 813b of the first frangible pathway 700a in a direction D2 that is generally laterally outward and longitudinally inward from the central region 106c of the first belt 106 and toward the first proximal terminus 810a in the first end region 106a of the first belt 106. Similar to the view shown in Figure 6C, Figure 7E shows a front plan view of the diaper pant of Figure 7C as the first frangible pathway 700a is being torn. As shown in Figure 7E, the first fastener part 707' is separated from the second fastener part 707" as the frangible pathway is torn.

As shown in Figure 6D, the first belt 106 may be separable along the first frangible pathway 700a to define a first belt zone 831. For example, the first belt zone 831 may be formed once the first tear line 705a propagates through the first distal terminus 808a and the second tear line 705b propagates through to the first proximal terminus 810a, the first belt zone 831 may be formed. As shown in Figure 6D, a first edge 831a of the first belt zone 831 is formed by tearing the first frangible pathway 700a. In addition, a first edge 833a of the third belt zone 833 discussed in more detail below is also formed by tearing the first frangible pathway 700a. The first belt zone 831 may extend from the first edge 831a of the first and second tear lines 705a, 705b to the first side seam 178 or the first longitudinal side edge 111a of the first belt 106. In addition, the first belt zone 831 may include the first fastener component 707a.

With the first belt zone 831 being defined by tearing the first belt 106 along the first frangible pathway 700a, a user may proceed to define the second belt zone 832 by tearing the first belt 106 along the second frangible pathway 700b. Referring now to Figures 6D and 6E, a caregiver may insert a finger or thumb through the second accessibility opening 802b and grasp the first belt 106 and the second fastener component 707b with a first hand. The caregiver's opposing second hand may be used to help stabilize the wearer. For example, the caregiver's opposing second hand may apply a holding or stabilizing force to the wearer at the central region 106c of the first belt 106. The user's first hand may then exert a pulling force F on the second grip region 801b of the first belt 106 outward away from the wearer to initiate a tearing of the first belt 106 along the second frangible pathway 700b, such as shown in Figure 6E.

With continued reference to Figure 6E, a force F generally represented by an arrow is applied to the second grip region 801b in a direction generally toward the second end region 106b of the first belt 106 and/or outward away from the first belt 106. As the force F is applied, a first tear line 705a and a second tear line 705b may simultaneously propagate along the first tear zone 813a and the second tear zone 813b, respectively. The first tear line 705a may propagate from the second accessibility opening 802b along the first tear zone 813a of the second frangible pathway 700b in longitudinal and lateral directions around the second fastener component 707b and then in a direction D1 that is generally laterally and longitudinally outward from the central region 106c of the first belt 106 and toward the second distal terminus 808b in the second end region 106b of the first belt 106. Simultaneously, the second tear line 705b may propagate from the second accessibility opening 802b along the second tear zone 813b of the second frangible pathway 700b in a direction D2 that is generally laterally outward and longitudinally inward from the central region 106c of the first belt 106 and toward the second proximal terminus 810b in the second end region 106b of the first belt 106.

As shown in Figure 6F, the first belt 106 may be separable along the second frangible pathway 700b to define a second belt zone 832 and a third belt zone 833. For example, the second belt zone 832 may be formed once the first tear line 705a propagates through the second distal terminus 808b and the second tear line 705b propagates through to the second proximal terminus 810b, the second belt zone 832 may be formed. As shown in Figure 6F, a first edge 832a of the second belt zone 832 is formed by tearing the second frangible pathway 700b. In addition, a second edge 833b of the third belt zone 833 is also formed by tearing the second frangible pathway 700b. The second belt zone 832 may extend from the first edge 832a of the first and second tear lines 705a, 705b to the second side seam 180 or the second longitudinal side edge 111b of the first belt 106. In addition, the second belt zone 832 may include the second fastener component 707b. The third belt zone 833 may extend laterally between the first edge 833a and the second edge 833b and may remain connected with the chassis 102. When completing the tearing operation on the configuration shown in Figures 7C-7E, the first belt zone 831 and the second belt zone 832 will include first fastener parts 707', and the third belt zone 833 will include second fastener parts 707" separated from respective first fastener parts 707' during the tearing of frangible pathways 700.

Although the tearing process is described above with reference to Figures 6A-6F as tearing the first belt 106 along the first frangible pathway 700a before tearing the first belt along the second frangible pathway 700b, it is to be appreciated that the tearing of first belt 106 along the frangible pathways 700 may occur in various different orders and in different manners. For example, the first belt 106 may be torn along second frangible pathway 700b to define the second belt zone 832 before tearing the first belt 106 along the first frangible pathway 700a to define the first belt zone 831. In another example, the first belt 106 may be torn simultaneously along the first frangible pathway 700a and the second frangible pathway 700b to define the first belt zone 831, the second belt zone 832, and the third belt zone 833.

Once the first belt 106 is torn along the frangible pathways 700 to define the first belt zone 831, the second belt zone 832, and the third belt zone 833, the diaper pant 100P may be removed from a wearer in a manner similar to that of a conventional taped diaper. After being removed from a wearer, the diaper pant 100P may be placed in a disposal configuration, such as discussed above with reference to Figures 5A and 5B, by rolling and/or folding the chassis 102 onto itself in a longitudinal direction. The first belt zone 831 and the second belt zone 832 may be used to further wrap the diaper pant 100P onto itself. And the fastener components 707 on the first belt zone 831 and the second belt zone 832 may be connected with another portion of the diaper pant 100P to help maintain the diaper pant 100P in the disposal configuration.

As shown in Figures 8A and 8B, an absorbent article may comprise one or more opening facilitation features 900 that may help a user successfully determine the location and/or function of the fastener component 707 and frangible pathway 700 and/or successfully complete pant opening operations. Such opening facilitation features 900 may comprise opening operational indicia 902 and opening mechanical aids 904, and combinations thereof. Figure 8A is a front plan view of a diaper pant 100P showing opening facilitation features 900 generically represented by shaded areas, and Figure 8B is a front plan view of a diaper pant 100P showing an example implementation of opening facilitation features 900.

As used herein, the term "opening operational indicium or indicia" generally refers to distinctive marks, colored regions, patterns, and/or textures disposed on a disposable absorbent article on and/or adjacent an accessibility opening 802, a fastener component 707, and/or a frangible pathway 700 to provide a functional attribute. Particularly, the functional attribute includes providing a visual and/or tactile indication that facilitates a relatively easy intuitive ability to locate an accessibility opening, a fastener component 707, and/or a frangible pathway 700 and/or separate a belt along frangible pathways 700 on the disposable absorbent article 100 during wearer removal and/or disposal.

Opening operational indicia 902 may include any visual or tactile differences or signals that enhance a user's ability to identify and/or use components in the diaper pant 100P removal and/or disposal process. All examples herein should be considered non-limiting. In some configurations, the opening operational indicia 902 may comprise visual and/or tactile indication of the location of the accessibility opening 802 and may be aligned with, or disposed in an adjacent or overlapping configuration with, the accessibility opening 802 along at least a portion of a length of the accessibility opening 802. In some configurations, an opening operational indicium 902 may be disposed on other portions of the first and/or second elastic belt 106, 108 or the backsheet 136 of the chassis 102 non-adjacent the accessibility opening 802 and may be accompanied by other opening operational indicia 902 cognitively connecting the indicis to the location of the accessibility opening 802. In some configurations, the opening operational indicia 902 may be disposed: adjacent to or across the accessibility opening 802 and/or frangible pathway 700, on the fastener component 707 or any material affixed to the garment-facing side of the fastener component 707; and/or on the first and/or second elastic belt 106, 108 or on the chassis 102. In some configurations, the opening operational indicia 902 may be formed by mechanically altering the fastener component 707 in such a way that forms or creates: relatively thinner and thicker portions; relatively higher and lower opacity regions; and/or physical holes, separations, and/or slits that may be adapted to allow a color or texture of materials directly beneath the fastener component 707 to be seen by a user, such as a caregiver.

In some configurations, a diaper pant 100P may comprise opening operational indicia 902 that comprise opening mechanical aid 906, which may be configured to help a user locate an accessibility opening 802. In some configurations, an accessibility opening indicium 906 may be printed on the first and/or second elastic belt 106, 108, the fastener component 707; and/or the backsheet 136 of the chassis 102. In some configurations, an accessibility opening indicium 906 may include graphics, such as for example: a linear or curvilinear representation of the accessibility opening 802; a geometric shape; a directional graphic such as arrows; anthropomorphic graphics such as hands, people and/or animals; alphanumeric text such as numerals, letters and/or words; and/or any combination thereof, and/or any other graphic that may indicate the location of the accessibility opening 802. The opening operational indicia 902 may also comprise one or more regions of the first and/or second belt 106, 108, or component materials having a texture or visually distinct area versus the remainder of the belt. For example, the garment-facing surface 117a, 117b of the first and/or second belt 106, 108, such as the first substrate 162 of the first and/or second belt 106, 108, may be mechanically deformed, bonded, and/or compressed to provide a visually distinct area that indicates to a user, such as a caregiver, the location of the accessibility opening 802.

In some configurations, a diaper pant 100P may comprise opening operational indicia 902 that comprise frangible pathway guides 908, which may be configured to help a user, such as a caregiver, discern where separation should occur and/or provide guidance as to the direction in which to pull. In some configurations, frangible pathway guides 908 may provide visual and/or tactile indication of the location of the frangible pathway 700 and may be aligned with and/or disposed in an adjacent and/or overlapping configuration with at least a portion of the frangible pathway 700. The frangible pathway guide 908 may indicate the location and direction of the frangible pathway 700 to help guide a user in the complete separation of the first and/or second belt 106, 108 along the frangible pathway 700. In some configurations, the frangible pathway guide 908 may be disposed on a portion of the first and/or second belt 106, 108 and/or backsheet 136 of the chassis 102. In some configurations, the frangible pathway guide 908 may comprise a printed linear and/or curvilinear representation of the frangible pathway 700 and/or may comprise directional graphics such as arrows and/or a series or array of relatively smaller graphics along the frangible pathway 700 indicating the direction in which the user may separate the frangible pathway 700. In some configurations, the frangible pathway guide 908 may comprise a portion of first and/or second belt materials or fibers of belt materials that are deformed, flattened, and/or mechanically altered in a way that changes either the opacity and/or topography of the belt material or belt fibers. In some configurations, the frangible pathway guide 908 may comprise more than one type of indicium in parallel or series along at least a portion of the frangible pathway 700.

In some configurations, opening operational indicia 902 may comprise a combination of one or more opening mechanical aid 906 and frangible pathway guides 908, which may be physically separate from one another and/or different in kind or type, and/or may represent portions of a relatively large opening operational indicia 902. For example, an accessibility opening indicium 906 may include a color, width, intensity, and/or style that may be different from a color, width, intensity, and/or style of the frangible pathway guide 908. In some configurations, the opening mechanical aid 904 may be visually distinct from and imply a temporal function order priority versus the frangible pathway guides 908. For example, a user's attention and action may be preferentially directed to the accessibility opening 802 to help enable the initiation of the pant opening, and secondarily, the frangible pathway guides 908 may indicate to the user the direction of the frangible pathway tear propagation to help maximize the probability of the user to successfully open the diaper pant 100P.

In some configurations, opening operation indicia 902 may comprise tinting of the fastener component material or other means of highlighting the location of the fastener component 707. For example, the fastener component 707 may be printed and/or comprise materials of various different colors such that the fastener component 707 may be externally visible from outside the diaper pant 100P. The fastener component 707 may have a color that is visible through any layers of the first and/or second belt 106, 108 on which the fastener component 707 is located. It is to be appreciated that the elastic belt 106, 108 and/or chassis 102 may include printing or other indicia highlighting to a user the location, function, and/or usage of the fastener component 707.

As used herein, the term "externally visible", as used in reference to an indicium associated with an article, refers to the ability of a human viewer to visually discern the indicium with the unaided eye (excepting standard corrective lenses adapted to compensate for nearsightedness, farsightedness, or astigmatism) in standard lighting conditions from a point of reference viewing the garment-facing surface of the article while the article is held in a configuration wherein the garment-facing surface is within the field of view. As used herein, "standard lighting conditions" refer to lighting conditions in which human vision operates efficiently (e.g., the human eye is able to discern complex patterns, shading, and colors). Specifically, standard lighting conditions may comprise at least one of the following: a) natural illumination as experienced outdoors during daylight hours, b) the illumination of a standard 100 watt incandescent white light bulb at a distance of 2 meters, or c) as defined by CIE D65 standard illuminate lighting at 800 lux to a 1964 CIE standard observer.

In some configurations, opening operational indicia 902 may comprise a bond or bond pattern attaching the fastener component 707 to the first and/or second belt 106, 108 that may be visually or tactilely distinct from the surrounding belt material. Such a bond or bond pattern may provide a user an indicium indicating the location of the fastener component 707 and/or a signal or a mechanical grip advantage. Additionally, the backsheet 136 of the chassis 102, such as a film layer, may be tinted or printed in a contrasting or complementary manner. A combination of tinting of the fastener component 707; fastener component-to-belt bond pattern; and/or backsheet printing may further accentuate the fastener component 707 location signal. In some configurations, the first elastic belt 106 comprise a region of a relatively low opacity to help enhance the visibility of any indicia or other printing disposed on the chassis 102.

It is to be appreciated that diaper pants 100P may be packaged for sale in either a physical or virtual store. As such, packages of the diaper pants 100P described herein may include artwork providing a user with an indication of the type of article enclosed therein, including the modes of removal and disposal features that may be enabled by the enclosed diaper pants. Pant removal and disposal features, such as fastener components 707, accessibility openings 802, and frangible pathways 700 and/or the way in which a user may use such features to remove a diaper pant 100P from a wearer and dispose of the diaper pant in a hygienic manner may be shown, demonstrated, and/or implied by artwork on the exterior of the package. In some configurations, artwork may include text, photos, drawings, and/or iconography. The artwork may indicate to users, such as caregivers, the usage mode of the articles within the package. For example, a package containing diaper pants described herein may depict images of a diaper pant being removed from a wearer by grasping the fastener and separating one or more frangible pathways 700 while the wearer is in a standing or laying position or may depict the changing process in a drawing or sketch form. In some configurations, a package containing diaper pants may comprise an icon showing an open pant and/or the process of application or removal of said pant. The artwork may additionally include a depiction of the disposal process, wherein a soiled diaper pant is rolled or folded, then wrapped within separated belt zones 831, 832 and fastened in such a configuration with the fastener components 707. In some configurations, advertising media may comprise artwork indicating the mode of removal and disposal of diaper pants described herein.

As discussed above, a diaper pant 100P may comprise one or more opening facilitation features 900 that may comprise opening mechanical aids 904. Opening mechanical aids 904 may comprise any property or feature that facilitates the ability of a user to physically access the fastener component 707 through the accessibility opening 802; securely grasp the fastener component 707; pull the fastener component 707 away from the chassis 102; and/or completely separate the frangible pathway 700 along an entire length thereof to open the diaper pant 100P and free the belt zones for use in secure closing of a rolled, soiled diaper pant 100P for a hygienic disposal. In some configurations, opening mechanical aids 904 may comprise aspects of the fastener component 707, such as for example: fastener component shape; fastener component physical properties such as thickness, stiffness, and/or coefficient of friction; and/or bonds connecting the fastener component 707 to the first and/or second belt 106, 108. In some configurations, opening mechanical aids 904 may comprise properties of the first and/or second belt 106, 108; the size, shape and/or orientation of the accessibility opening 802; and/or any separate elements attached to the first and/or second belt 106, 108 or fastener component 707. Opening mechanical aids 904 may also include tactile properties, such as for example, physical bending of portions of the fastener component 707 to help facilitate both visual noticeability and location of the fastener component 707.

In some configurations, opening mechanical aids 904 may comprise a fastener component 707 having a three-dimensional aspect. For example, such a three-dimensional aspect may comprise a configuration in which a portion of the fastener component 707 bends or curls away from the plane of the fastener component 707 and/or the plane of the first and/or second belt 106, 108 in a direction away from the wearer. A fastener component 707 having a three-dimensional aspect may be more easily grasped by a user. For example, if a portion of the accessibility opening 802 becomes folded into, partially hidden, or otherwise located inside a wrinkle formed while the diaper pant 100P is being worn, the three-dimensional aspect may provide a location to help aid a user to grasp the fastener component 707. In some configurations, the fastener component 707 may extend from about 1mm to about 5 mm above a plane of the first and/or second belt 106, 108. In some configurations, a length of the three-dimensional aspect may be from about 5 mm to about 20 mm. It is to be appreciated that a three-dimensional aspect may be formed in the fastener component 707 in various ways, such as by deforming the fastener component material by bonding the material or advancing the material over a curved surface while under tension.

In configurations wherein the opening mechanical aid is intended to facilitate grasping and pulling of the fastener component 707 away from the chassis 102 and separating completely the frangible pathway 700, the opening mechanical aid 904 may comprise three-dimensional features and/or a relatively higher coefficient of friction on at least a portion of material of the fastener component 707 or material of first and/or second belt material to help provide a user a relatively more secure grip on the fastener component 707 and/or a mechanical advantage to help successfully pull the fastener component 707 to open the diaper pant 100P. Three-dimensional features may be provided on the fastener component 707 and/or the first and/or second belts 106, 108 in various ways, such as for example, by a three-dimensional bonding means and pattern of the fastener to the elastic belt, such as a thermal, ultrasonic or mechanical bond pattern. In some configurations, the fastener component 707 may be bonded completely through the thickness of the fastener component 707 from, for example, the inner surface of the hooks, through the fastener material, and then to the inner surface material of the first and/or second belt 106, 108. In some configurations, a bonding process may proceed from the outer surface of the first and/or second belt 106, 108, through the thickness of the belt, and then into the fastener component 707. In both of such configurations, a three-dimensional mechanical opening aid may be provided via the bond geometry and/or texture. In some configurations, an opening mechanical aid 904 may be provided by the deposition of a material, such as a molten polymer or adhesive, onto the fastener component 707 or the first and/or second belt 106, 108, such as for example, in a region comprising the fastener component 707 or in an adjacent region. In some configurations, a separate material piece may be connected to the first and/or second belt in a region of the fastener component 707 or an adjacent region by various means known in the art.

### AVERAGE DECITEX (AVERAGE-DTEX)

The Average Decitex Method is used to calculate the Average-Dtex on a length-weighted basis for elastic fibers present in an entire article, or in a specimen of interest extracted from an article. The decitex value is the mass in grams of a fiber present in 10,000 meters of that material in the relaxed state. The decitex value of elastic fibers or elastic laminates containing elastic fibers is often reported by manufacturers as part of a specification for an elastic fiber or an elastic laminate including elastic fibers. The Average-Dtex is to be calculated from these specifications if available. Alternatively, if these specified values are not known, the decitex value of an individual elastic fiber is measured by determining the cross-sectional area of a fiber in a relaxed state via a suitable microscopy technique such as scanning electron microscopy (SEM), determining the composition of the fiber via Fourier Transform Infrared (FT-IR) spectroscopy, and then using a literature value for density of the composition to calculate the mass in grams of the fiber present in 10,000 meters of the fiber. The manufacturer-provided or experimentally measured decitex values for the individual elastic fibers removed from an entire article, or specimen extracted from an article, are used in the expression below in which the length-weighted average of decitex value among elastic fibers present is determined.

The lengths of elastic fibers present in an article or specimen extracted from an article is calculated from overall dimensions of and the elastic fiber pre-strain ratio associated with components of the article with these or the specimen, respectively, if known. Alternatively, dimensions and/or elastic fiber pre-strain ratios are not known, an absorbent article or specimen extracted from an absorbent article is disassembled and all elastic fibers are removed. This disassembly can be done, for example, with gentle heating to soften adhesives, with a cryogenic spray (e.g., Quick-Freeze, Miller-Stephenson Company, Danbury, CT), or with an appropriate solvent that will remove adhesive but not swell, alter, or destroy elastic fibers. The length of each elastic fiber in its relaxed state is measured and recorded in millimeters (mm) to the nearest mm.

### Calculation of Average-Dtex

For each of the individual elastic fibers *fᵢ* of relaxed length *Lᵢ* and fiber decitex value *dᵢ* (obtained either from the manufacturer's specifications or measured experimentally) present in an absorbent article, or specimen extracted from an absorbent article, the Average-Dtex for that absorbent article or specimen extracted from an absorbent article is defined as: $Average - Dtex = \frac{{\sum }_{i = 1}^{n} \left({L}_{i}\times {d}_{i}\right)}{{\sum }_{i = 1}^{n} {L}_{i}}$ where n is the total number of elastic fibers present in an absorbent article or specimen extracted from an absorbent article. The Average-Dtex is reported to the nearest integer value of decitex (grams per 10 000 m).

If the decitex value of any individual fiber is not known from specifications, it is experimentally determined as described below, and the resulting fiber decitex value(s) are used in the above equation to determine Average-Dtex.

### Experimental Determination of Decitex Value for a Fiber

For each of the elastic fibers removed from an absorbent article or specimen extracted from an absorbent article according to the procedure described above, the length of each elastic fiber *Lₖ* in its relaxed state is measured and recorded in millimeters (mm) to the nearest mm. Each elastic fiber is analyzed via FT-IR spectroscopy to determine its composition, and its density *ρₖ* is determined from available literature values. Finally, each fiber is analyzed via SEM. The fiber is cut in three approximately equal locations perpendicularly along its length with a sharp blade to create a clean cross-section for SEM analysis. Three fiber segments with these cross sections exposed are mounted on an SEM sample holder in a relaxed state, sputter coated with gold, introduced into an SEM for analysis, and imaged at a resolution sufficient to clearly elucidate fiber cross sections. Fiber cross sections are oriented as perpendicular as possible to the detector to minimize any oblique distortion in the measured cross sections. Fiber cross sections may vary in shape, and some fibers may consist of a plurality of individual filaments. Regardless, the area of each of the three fiber cross sections is determined (for example, using diameters for round fibers, major and minor axes for elliptical fibers, and image analysis for more complicated shapes), and the average of the three areas *aₖ* for the elastic fiber, in units of micrometers squared (µm²), is recorded to the nearest 0.1 µm². The decitex *dₖ* of the kth elastic fiber measured is calculated by: ${d}_{k} = 10 000 m \times {a}_{k} \times {ρ}_{k} \times {10}^{- 6}$ where *dₖ* is in units of grams (per calculated 10,000 meter length), *aₖ* is in units of µm², and *ρₖ* is in units of grams per cubic centimeter (g/cm³). For any elastic fiber analyzed, the experimentally determined *Lₖ* and *dₖ* values are subsequently used in the expression above for Average-Dtex.

### AVERAGE-STRAND-SPACING

Using a ruler calibrated against a certified NIST ruler and accurate to 0.5 mm, measure the distance between the two distal strands within a section to the nearest 0.5 mm, and then divide by the number of strands in that section - 1 $Average - Strand - Spacing = d / \left(n-1\right)$ where n>1
report to the nearest 0.1 mm.

### AVERAGE-PRE-STRAIN

The Average-Pre-Strain of a specimen are measured on a constant rate of extension tensile tester (a suitable instrument is the MTS Insight using Testworks 4.0 Software, as available from MTS Systems Corp., Eden Prairie, MN) using a load cell for which the forces measured are within 1% to 90% of the limit of the cell. Articles are conditioned at 23 °C ± 2 C° and 50% ± 2% relative humidity for 2 hours prior to analysis and then tested under the same environmental conditions.

Program the tensile tester to perform an elongation to break after an initial gage length adjustment. First raise the cross head at 10 mm/min up to a force of 0.05N. Set the current gage to the adjusted gage length. Raise the crosshead at a rate of 100 mm/min until the specimen breaks (force drops 20% after maximum peak force). Return the cross head to its original position. Force and extension data is acquired at a rate of 100 Hz throughout the experiment.

Set the nominal gage length to 40 mm using a calibrated caliper block and zero the crosshead. Insert the specimen into the upper grip such that the middle of the test strip is positioned 20 mm below the grip. The specimen may be folded perpendicular to the pull axis, and placed in the grip to achieve this position. After the grip is closed the excess material can be trimmed. Insert the specimen into the lower grips and close. Once again, the strip can be folded, and then trimmed after the grip is closed. Zero the load cell. The specimen should have a minimal slack but less than 0.05 N of force on the load cell. Start the test program.

From the data construct a Force (N) verses Extension (mm). The Average-Pre-Strain is calculated from the bend in the curve corresponding to the extension at which the nonwovens in the elastic are engaged. Plot two lines, corresponding to the region of the curve before the bend (primarily the elastics), and the region after the bend (primarily the nonwovens). Read the extension at which these two lines intersect, and calculate the % Pre-Strain from the extension and the corrected gage length. Record as % Pre-strain 0.1%. Calculate the arithmetic mean of three replicate samples for each elastomeric laminate and Average-Pre-Strain to the nearest 0.1%.

### Bio-Based Content for Components

Components of the absorbent articles described herein may at least partially be comprised of bio-based content as described in U.S. Pat. Appl. No. 2007/0219521A1. For example, the superabsorbent polymer component may be bio-based via their derivation from bio-based acrylic acid. Bio-based acrylic acid and methods of production are further described in U.S. Pat. Appl. Pub. No. 2007/0219521 and U.S. Pat. Nos. 8,703,450; 9,630,901 and 9,822,197. Other components, for example nonwoven and film components, may comprise bio-based polyolefin materials. Bio-based polyolefins are further discussed in U.S. Pat. Appl. Pub. Nos. 2011/0139657, 2011/0139658, 2011/0152812, and 2016/0206774, and U.S. Pat. No. 9,169,366. Example bio-based polyolefins for use in the present disclosure comprise polymers available under the designations SHA7260^{™}, SHE150^{™}, or SGM9450F^{™} (all available from Braskem S.A.).

An absorbent article component may comprise a bio-based content value from about 10% to about 100%, from about 25% to about 100%, from about 40% to about 100%, from about 50% to about 100%, from about 75% to about 100%, or from about 90% to about 100%, for example, using ASTM D6866-10, method B.

### Recycle Friendly and Bio-Based Absorbent Articles

Components of the absorbent articles described herein may be recycled for other uses, whether they are formed, at least in part, from recyclable materials. Examples of absorbent article materials that may be recycled are nonwovens, films, fluff pulp, and superabsorbent polymers. The recycling process may use an autoclave for sterilizing the absorbent articles, after which the absorbent articles may be shredded and separated into different byproduct streams. Example byproduct streams may comprise plastic, superabsorbent polymer, and cellulose fiber, such as pulp. These byproduct streams may be used in the production of fertilizers, plastic articles of manufacture, paper products, viscose, construction materials, absorbent pads for pets or on hospital beds, and/or for other uses. Further details regarding absorbent articles that aid in recycling, designs of recycle friendly diapers, and designs of recycle friendly and bio-based component diapers, are disclosed in U.S. Pat. Appl. Publ. No. 2019/0192723, published on June 27, 2019.

## Claims

1. An absorbent article comprising:
a first belt (106) comprising an inner wearer facing surface (115b) and an outer garment facing surface (115a), the first belt (106) further comprising a laterally extending inner edge (107b) and a laterally extending outer edge (107a), the outer edge (107a) positioned longitudinally outward of the inner edge (107b);
a second belt (108), wherein laterally opposing end portions (108a, 108b) of the second belt (108) are connected with laterally opposing end portions (106a, 106b) of the first belt (106) at a first side seam (178) and a second side seam (180) to form a waist opening (110);
a chassis (102) comprising a topsheet (138), a backsheet (136), and an absorbent core (140) positioned between the topsheet (138) and the backsheet (136), the chassis (102) comprising a longitudinally extending first side edge (128) and a longitudinally extending second side edge (130) laterally separated from the first side edge (128) by a first end edge (144) and a second end edge (146) longitudinally separated from the first end edge (144), and wherein longitudinally opposing end regions (116a, 118a) of the chassis (102) are connected with the first belt (106) and the second belt (108);
wherein a portion of the chassis (102) overlaps the inner wearer facing surface (115b) of the first belt (106) to define a chassis overlap region (850);
a frangible pathway (700) in the first belt (106) extending across the overlap region (850) between a proximal terminus (810) on the inner edge (107b) and a distal terminus (808) on the outer edge (107a) of the first belt (116), the frangible pathway (700) comprising a first tear zone (813a) extending from the overlap region (850) to the distal terminus (808), and a second tear zone (813b) extending from the overlap region (850) to the proximal terminus (810); and
a fastener component (707) on the inner wearer facing surface (115b) of the first belt (106) in the overlap region (850); and
an accessibility opening (802) in the first belt (106) in the overlap region (850) positioned between the first tear zone (813a) and the inner edge (107b) of the first belt (107a), wherein the accessibility opening (802) comprises a slit in the first belt (106).

2. The absorbent article of claim 1, wherein the distal terminus (808) and the proximal terminus (810) are positioned laterally between the first side edge (128) of the chassis (102) and the first side seam (178).

3. The absorbent article of either claim 1 or 2, wherein the first tear zone extends (813a) from the accessibility opening (802) to the distal terminus (808), and the second tear zone (813b) extends from the accessibility opening (802) to the proximal terminus (810).

4. The absorbent article of claim 1, wherein the slit extends along a line in a lateral direction to define a length of about 5 mm to about 50 mm and an angle from about 0 degrees to about 10 degrees with respect to the inner edge (107b) of the first belt (106).

5. The absorbent article of any of the preceding claims, wherein the first belt (106) is separable along the frangible pathway (700) to define a first belt zone (831) and a second belt zone (833).

6. The absorbent article of claim 5, wherein the fastener component (707) is adapted to refastenably connect the first belt zone (831) with at least one other component of the absorbent article in a disposal configuration.

7. The absorbent article of any of the preceding claims, wherein the fastener component (707) comprises hooks (715), and the wherein the hooks (715) extend from the first belt (106) and toward the b acksheet (136).

8. The absorbent article of any of the preceding claims, wherein the backsheet (136) comprises a laminate comprising a nonwoven layer and a film layer.

9. The absorbent article of claim 7, wherein the hooks (715) are in direct contact with the film layer and not refastenably connected with the film layer.

10. The absorbent article of any of the preceding claims, further comprising an opening operational indicium (902) adapted to at least one of: facilitate location of the accessibility opening (802) and indicate proper separation of the first belt (106) along the frangible pathway (700).

## Patentansprüche

1. Absorptionsartikel, umfassend:
ein erstes Band (106), umfassend eine trägerseitige Innenoberfläche (115b) und eine bekleidungsseitige Außenoberfläche (115a), das erste Band (106) ferner umfassend einen sich seitlich erstreckenden Innenrand (107b) und einen sich seitlich erstreckenden Außenrand (107a), wobei der Außenrand (107a) längs außerhalb des Innenrands (107b) angeordnet ist;
ein zweites Band (108), wobei seitlich entgegengesetzte Endabschnitte (108a, 108b) des zweiten Bands (108) mit seitlich entgegengesetzten Endabschnitten (106a, 106b) des ersten Bands (106) an einer ersten Seitennaht (178) und einer zweiten Seitennaht (180) verbunden sind, um eine Taillenöffnung (110) auszubilden;
eine Grundeinheit (102), umfassend eine Oberschicht (138), eine Unterschicht (136) und einen Absorptionskern (140), der zwischen der Oberschicht (138) und der Unterschicht (136) angeordnet ist, die Grundeinheit (102) umfassend einen sich längs erstreckenden ersten Seitenrand (128) und einen sich längs erstreckenden zweiten Seitenrand (130), der seitlich von dem ersten Seitenrand (128) durch einen ersten Endrand (144) und einen zweiten Endrand (146) getrennt ist, der längs von dem ersten Endrand (144) getrennt ist, und wobei längs entgegengesetzte Endbereiche (116a, 118a) der Grundeinheit (102) mit dem ersten Band (106) und dem zweiten Band (108) verbunden sind;
wobei ein Abschnitt der Grundeinheit (102) die trägerseitige Innenoberfläche (115b) des ersten Bands (106) überlappt, um einen Überlappungsbereich (850) der Grundeinheit zu definieren;
einen reißbaren Pfad (700) in dem ersten Band (106), der sich über den Überlappungsbereich (850) zwischen einem nahe gelegenen Endpunkt (810) auf dem Innenrand (107b) und einem distalen Endpunkt (808) auf dem Außenrand (107a) des ersten Bands (116) erstreckt, der reißbare Pfad (700) umfassend einen ersten Rissbereich (813a), der sich von dem Überlappungsbereich (850) zu dem distalen Endpunkt (808) erstreckt, und einen zweiten Rissbereich (813b), der sich von dem Überlappungsbereich (850) zu dem nahe gelegenen Endpunkt (810) erstreckt; und
einen Befestigungsmittelbestandteil (707) auf der trägerseitigen Innenoberfläche (115b) des ersten Bands (106) in dem Überlappungsbereich (850); und
eine Zugänglichkeitsöffnung (802) in dem ersten Band (106) in dem Überlappungsbereich (850), die zwischen dem ersten Rissbereich (813a) und dem Innenrand (107b) des ersten Bands (107a) angeordnet ist, wobei die Zugänglichkeitsöffnung (802) einen Schlitz in dem ersten Band (106) umfasst.

2. Absorptionsartikel nach Anspruch 1, wobei der distale Endpunkt (808) und der nahe gelegene Endpunkt (810) seitlich zwischen dem ersten Seitenrand (128) der Grundeinheit (102) und der ersten Seitennaht (178) angeordnet sind.

3. Absorptionsartikel nach Anspruch 1 oder 2, wobei sich der erste Rissbereich (813a) von der Zugänglichkeitsöffnung (802) zu dem distalen Endpunkt (808) erstreckt und sich der zweite Rissbereich (813b) von der Zugänglichkeitsöffnung (802) zu dem nahe gelegenen Endpunkt (810) erstreckt.

4. Absorptionsartikel nach Anspruch 1, wobei sich der Schlitz entlang einer Linie in einer Querrichtung erstreckt, um eine Länge von etwa 5 mm bis etwa 50 mm und einen Winkel von etwa 0 Grad bis etwa 10 Grad in Bezug auf den Innenrand (107b) des ersten Bands (106) zu definieren.

5. Absorptionsartikel nach einem der vorstehenden Ansprüche, wobei das erste Band (106) entlang des reißbaren Pfads (700) trennbar ist, um einen ersten Bandbereich (831) und einen zweiten Bandbereich (833) zu definieren.

6. Absorptionsartikel nach Anspruch 5, wobei der Befestigungsmittelbestandteil (707) konzipiert ist, um den ersten Bandbereich (831) mit wenigstens einem anderen Bestandteil des Absorptionsartikels in einer Einwegkonfiguration wiederbefestigbar zu verbinden.

7. Absorptionsartikel nach einem der vorstehenden Ansprüche, wobei der Befestigungsmittelbestandteil (707) Haken (715) umfasst und wobei sich die Haken (715) von dem ersten Band (106) und zu der Unterschicht (136) hin erstrecken.

8. Absorptionsartikel nach einem der vorstehenden Ansprüche, wobei die Unterschicht (136) ein Laminat, umfassend eine Vliesschicht und eine Folienschicht, umfasst.

9. Absorptionsartikel nach Anspruch 7, wobei die Haken (715) in direktem Kontakt mit der Folienschicht stehen und mit der Folienschicht nicht wiederbefestigbar verbunden sind.

10. Absorptionsartikel nach einem der vorstehenden Ansprüche, ferner umfassend ein Zeichen (902) für einen Öffnungsablauf, das konzipiert ist zu wenigstens einem von: Ermöglichen einer Lokalisierung der Zugänglichkeitsöffnung (802) und Anzeigen einer sachgerechten Trennung des ersten Bands (106) entlang des reißbaren Pfads (700).

## Revendications

1. Article absorbant comprenant :
une première ceinture (106) comprenant une surface interne faisant face au porteur (115b) et une surface externe faisant face au vêtement (115a), la première ceinture (106) comprenant en outre un bord interne s'étendant latéralement (107b) et un bord externe s'étendant latéralement (107a), le bord externe (107a) étant positionné longitudinalement vers l'extérieur du bord interne (107b) ;
une seconde ceinture (108), dans lequel des parties d'extrémité latéralement opposées (108a, 108b) de la seconde ceinture (108) sont reliées à des parties d'extrémité latéralement opposées (106a, 106b) de la première ceinture (106) au niveau d'une première couture latérale (178) et d'une seconde couture latérale (180) pour former une ouverture de taille (110) ;
un châssis (102) comprenant une feuille de dessus (138), une feuille de fond (136), et une âme absorbante (140) positionnée entre la feuille de dessus (138) et la feuille de fond (136), le châssis (102) comprenant un premier bord latéral s'étendant longitudinalement (128) et un second bord latéral s'étendant longitudinalement (130) séparé latéralement du premier bord latéral (128) par un premier bord d'extrémité (144) et un second bord d'extrémité (146) longitudinalement séparé du premier bord d'extrémité (144), et dans lequel des régions d'extrémité longitudinalement opposées (116a, 118a) du châssis (102) sont reliées à la première ceinture (106) et à la seconde ceinture (108) ;
dans lequel une partie du châssis (102) chevauche la surface interne faisant face au porteur (115b) de la première ceinture (106) pour définir une région de chevauchement de châssis (850) ;
une ligne de rupture (700) dans la première ceinture (106) s'étendant à travers la région de chevauchement (850) entre une partie terminale proximale (810) sur le bord interne (107b) et une partie terminale distale (808) sur le bord externe (107a) de la première ceinture (116), la ligne de rupture (700) comprenant une première zone de déchirure (813a) s'étendant de la région de chevauchement (850) à la partie terminale distale (808), et une seconde zone de déchirure (813b) s'étendant de la région de chevauchement (850) à la partie terminale proximale (810) ; et
un composant de fixation (707) sur la surface interne faisant face au porteur (115b) de la première ceinture (106) dans la région de chevauchement (850) ; et
une ouverture d'accessibilité (802) dans la première ceinture (106) dans la région de chevauchement (850) positionnée entre la première zone de déchirure (813a) et le bord interne (107b) de la première ceinture (107a), dans lequel l'ouverture d'accessibilité (802) comprend une fente dans la première ceinture (106).

2. Article absorbant selon la revendication 1, dans lequel la partie terminale distale (808) et la partie terminale proximale (810) sont positionnées latéralement entre le premier bord latéral (128) du châssis (102) et la première couture latérale (178).

3. Article absorbant selon la revendication 1 ou 2, dans lequel la première zone de déchirure s'étend (813a) de l'ouverture d'accessibilité (802) à la partie terminale distale (808), et la seconde zone de déchirure (813b) s'étend de l'ouverture d'accessibilité (802) à la partie terminale proximale (810).

4. Article absorbant selon la revendication 1, dans lequel la fente s'étend le long d'une ligne dans une direction latérale pour définir une longueur d'environ 5 mm à environ 50 mm et un angle d'environ 0 degré à environ 10 degrés par rapport au bord interne (107b) de la première ceinture (106).

5. Article absorbant selon l'une quelconque des revendications précédentes, dans lequel la première ceinture (106) est séparable le long de la ligne de rupture (700) pour définir une première zone de ceinture (831) et une seconde zone de ceinture (833).

6. Article absorbant selon la revendication 5, dans lequel le composant de fixation (707) est adapté pour relier de manière rattachable la première zone de ceinture (831) à au moins un autre composant de l'article absorbant dans une configuration de mise au rebut.

7. Article absorbant selon l'une quelconque des revendications précédentes, dans lequel le composant de fixation (707) comprend des crochets (715), et dans lequel les crochets (715) s'étendent depuis la première ceinture (106) et vers la feuille de fond (136).

8. Article absorbant selon l'une quelconque des revendications précédentes, dans lequel la feuille de fond (136) comprend un stratifié comprenant une couche de non-tissé et une couche de film.

9. Article absorbant selon la revendication 7, dans lequel les crochets (715) sont en contact direct avec la couche de film et ne sont pas reliés de manière rattachable à la couche de film.

10. Article absorbant selon l'une quelconque des revendications précédentes, comprenant en outre un indice opérationnel d'ouverture (902) adapté pour au moins l'un parmi : faciliter la localisation de l'ouverture d'accessibilité (802) et indiquer une séparation correcte de la première ceinture (106) le long de la ligne de rupture (700).
